(19) Europäisches Patentamt / European Patent Office / Office européen des brevets

(11) EP 1 721 598 B1

(12) EUROPEAN PATENT SPECIFICATION

(45) Date of publication and mention of the grant of the patent:
**28.09.2011 Bulletin 2011/39**

(51) Int Cl.:
***A61K 8/14*** (2006.01) ***A61Q 5/08*** (2006.01)
***A61Q 5/10*** (2006.01) ***A61Q 5/06*** (2006.01)

(21) Application number: **05009413.5**

(22) Date of filing: **29.04.2005**

(54) **Micelle thickening systems for hair colourant and bleaching compositions**

Mizell-Eindickungssysteme für Haarfärbe- und Haarbleichmittel

Système de micelles épaississant pour des compositions de coloration ou de blanchiment des cheveux

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**15.11.2006 Bulletin 2006/46**

(73) Proprietor: **THE PROCTER & GAMBLE COMPANY**
**Cincinnati, OH 45202 (US)**

(72) Inventors:
- **Bureiko, Andrei Sergeevich**
  **Sunningdale**
  **Berkshire**
  **SL5 OBB (GB)**
- **Glenn, Robert Wayne**
  **Surrey**
  **GU15 3DL (GB)**
- **McMeekin, Anthony NMN**
  **Bagshot**
  **Surrey**
  **GU19 5QY (GB)**
- **McKie, Ross Angus**
  **Ayrshire**
  **Scotland KA15 1HJ (GB)**

(74) Representative: **Kohol, Sonia et al**
**Procter & Gamble Technical Centres Limited**
**Patent Department**
**Rusham Park**
**Whitehall Lane**
**Egham**
**Surrey TW20 9NW (GB)**

(56) References cited:
**EP-A- 0 530 708**
**WO-A-01/28508**
**US-A1- 2003 073 606**
**US-A1- 2004 053 797**
**US-B1- 6 268 324**

- **W.G.MILLER ET.AL.: "Viscoelastic Micellar Solutions: Microscopy and Rheology" JOURNAL OF PHYSICAL CHEMISTRY, vol. 96, 1992, pages 474-484, XP002368280**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

FIELD OF THE INVENTION

The present invention relates to thickened hair colouring and or hair bleaching compositions.

BACKGROUND OF THE INVENTION

The permanent alteration of the colour of keratinous fibres, in particular human hair, by the application of hair dyes is well known. In order to provide the consumer with the hair colour and the intensity of colour desired, a very complex chemical process is utilized. Permanent hair dyeing formulations typically comprise oxidative hair dye precursors, which can diffuse into the hair through the cuticle and into the cortex where they can then react with each other and suitable oxidising agents to form the end dye molecules. Due to the larger size of these resultant molecules they are unable to readily diffuse out of the hair during subsequent washing with water and/or detergents; hence delivering a consumer-desired permanency of colour. This reaction typically takes place in an aggressive environment at approximately pH 10 in the presence of an alkalizing agent and in the presence of an oxidizing agent. Moreover, the consumer repeats this process regularly in order to maintain the desired hair colour and shade and the intensity of colour and to ensure continual, even coverage of the hair including coverage of new hair growth.

The manufacturer of such products is also required to work within a large number of constraints. Since these products are being placed in direct contact with the consumers' skin, the potential exists for accidental contact with the eye or for ingestion (for example), which can occur during the dyeing process. Therefore, the formulation must meet rigorous safety requirements and not induce any allergic reactions. In addition to meeting these requirements, the products must also be optically and olfactory pleasing to the consumer. In particular, the products also need to meet certain physical parameters in order to ensure that the product can be easily applied to the hair by the consumer to provide the desired effect, without unintentional staining of the consumers' clothes, skin or other objects.

The manufacturer is also required to provide the hair colouring consumer a large range of different resulting colours. Some consumers may just wish to enhance the natural colour of the hair, whilst others may wish to cover grey or completely alter the hair colour to a different natural appearing hair colour or a 'synthetic' appearing hair colour. Consequently, the manufacturer may provide over twenty different formulations, of varying colours and shades, to address the range of consumer specific needs. These formulations have to be individually formulated and are typically complex formulae containing a mixture of different dye compounds. As a result the manufacture of such product ranges can be costly and complex.

Typically permanent hair dye products will contain a source of alkali such as an ammonia source. This serves the purpose of swelling the hair allowing the entry of the dye precursor molecules into the hair and also improves the lightening effect of the oxidising agent, which is typically hydrogen peroxide. However, ammonia is also volatile and its associated odour is extremely unpleasant to the consumers' of such products, particularly as these hair dye products are used in close proximity to the nasal region. Hence, it would be highly desirable to provide an oxidative hair colouring and/or bleaching composition, which delivers the consumer required lightening level and colour, but which has reduced or eliminated the detectable ammonia odour.

In fact another deficiency area in current hair colouring products is the provision of hair colouring products which deliver the required hair lightening effect. Delivering the required level of lightening is particularly important in order to provide the full range of colour shades demanded by the consumer, especially for blonde shades and grey coverage. Such products pose particular difficulties to the manufacturer, as they usually require the use of high levels of oxidising agent and ammonia in order to deliver the required lightening effect. However, in additional to the problems associated with the presence of high levels of ammonia in these products, as discussed herein above, the presence of these high levels of ammonia and/or oxidizing agent also affect the condition of the hair and may in some cases induce mild skin irritation on the scalp. In particular, the hydrophilicity of the hair surface is increased during the colouring process, which alters the sensory perception of the hair and its overall manageability during, and immediately after colouring and during the subsequent wash and styling cycles until the next colourant application. Hence, it would also be highly desirable to provide an oxidative hair colouring and/or bleaching composition which delivers the required lightening and/or colour without unnecessary hair damage.

A number of attempts have been described in the literature to address at least some of the above identified improvement areas. For example the use of carbonate has been described in the following hair colouring art.

EP 435 012 describes hair-dyeing compositions, which require a short dyeing time, create little damage to hair, and no irritating odour after dyeing comprising a carbonate source, a non odour generating alkali hydrogen peroxide and a buffer solution. Similarly EP 1 106 166 describes hair dye compositions comprising ammonia, carbonate (other than ammonia salt), transition metal salt and chelating agent which do not give off an irritating odour, have low skin irritation and can change the hair colour into a lighter tone in a short time. WOO 1/28508 describes hair colouring

formulations comprising oxidising agents and ammonia carbonate or carbamate which deliver improved bleaching and colouring with reduced odour and hair damage without the need for buffering agents, pH modifiers or hair swelling agents. JPO1206825 describes a low pungent hair colouring composition comprising ammonia, ammonium salt and carbonate. US2004/0083557 describes hair colouring compositions comprising an oxidative hair dye precursor, a metal cyanate, an alkalizing agent and an oxidizing agent and preferably a metal bicarbonate salt in order to provide good colour lift and low odour.

WO04/014328 describes one step hair colouring compositions comprising peroxide oxidizing agents, specific oxidizing agents and at least one water soluble carbonate releasing salts which more effectively deliver colour wherein the composition is applied for a period of from 2 to 60 minutes. US2004/0098814 describes a method of permanently colouring hair whereby the hair is subjected to a number of consecutive short treatments whereby the treatment comprises a dye intermediate in a shampoo or conditioner base, a water soluble carbonate releasing salt and a water soluble ammonium salt. US2004/0098816 also describes a method for the gradual permanent colouring of hair which includes subjecting the hair to a number of treatments having a set time interval between them, wherein the treatment compositions comprise ammonium carbonate in combination with a chelant.

EP1484047 and EP148447 describe hair colouring compositions comprising a source of carbonate ions and oxidizing agents and a source of radical scavengers to provide improved hair colouring without odour and hair damage.

It has now however been found that the incorporation of hydrogen peroxide and carbonate hair colourant systems, results in difficulties in manufacturing such products. This problem is particularly manifest for hair colouring compositions which have high levels of peroxide and carbonate which are desirable to provide high levels of lift. In order to provide a product which the consumer can easily apply to the hair without dripping onto the skin, eyes, clothes or bathroom surfaces, hair colourant products are designed such that the composition applied on head has a certain required viscosity. This is either achieved by providing the dye composition and the oxidizing composition as so called 'thin-thin-thick' type liquid formulations which are thickened upon mixing. Alternatively, at least one of the components, either the dye composition or the oxidizing composition, preferably the dye composition, is provided as a thickened formulation which thickens the total composition upon mixing, which is a so called 'thick-thin-thick' formulation. Finally, the desired viscosity may also be provided by the use of so called 'thick-thick-thick' formulations wherein both the dye composition and the oxidizing compositions are provided as thickened formulations which upon mixing form a thickened total composition.

The above described viscosity manipulation can typically be achieved by the use of high concentrations of solvent systems. However, such solvent systems are not desirable in terms of skin mildness. Alternatively, thickening systems based upon polymers as described for example in EP 1047375 may be used. These systems purport to provide consumer acceptable rheology to the product such that it can be easily applied to the hair whilst importantly not dripping onto the skin or eyes during treatment. However, these materials have also been found not to sufficiently thicken compositions comprising high levels of carbonate resulting in product instability or unsatisfactory viscosity. Moreover, many of these system do not allow easy mixing of the components by the consumer resulting in inhomogeneous mixtures. Hence it would be desirable to provide a hair colorant composition which incorporates high levels of carbonate or indeed any other ions without compromising the product stability or ease of manufacture.

Another particularly critical performance area for the consumer is the provision of the desired resultant colour and also the effective coverage of grey hair. Indeed, whilst the amount of grey hair to be coloured varies considerably from consumer to consumer, the resultant overall appearance of the coloured hair demanded by the consumer should be nearly identical for the naturally pigmented hair and the grey hair on head, with the added requirement that the initial coverage is maintained during the post dyeing washing and drying cycle. In order to effectively incorporate the dyes into the compositions, again, typically high levels of solvent are required. However as discussed hereinabove this is not desirable.

Hence, it would be further desirable to provide the consumer with a hair colourant, providing improved lift and lightening and improved colour delivery, uptake and durability and which is easy to manufacture, delivering the required viscosity without the need for high solvent levels and which is shelf life stable.

It has now been surprisingly found that oxidative hair colouring compositions comprising an oxidizing agent, a worm-like micelle phase thickening system comprising an ionic surfactant and a specific level of counter ion concentration can be formulated as stable thickened systems which can be utilized in 'thin-thin-thin'systems, 'thin-thick-thick' systems and 'thick-thick-thick' systems. Moreover, the compositions of the present invention are compatible with current dyes and dye precursor systems and result in excellent dye deposition and colour and improved grey coverage. In addition, the compositions exhibit low odour and deliver a high level of lift and lightening equal to the currently utilised ammonia/ peroxide systems, whilst reducing the concentration of peroxide and reducing the hair fibre damage.

Worm-like micelle phase thickening systems have been described in non-hair color literature such as hard surface cleaning and laundry detergent compositions. For example viscoelastic cleansing gel with micellar surfactant solutions are described in US20040053797A1, liquid laundry detergent compositions are described in EP0530708A and thickened hard surface cleaners are discussed in EP070160B1. However, none of these documents describe the specific

micellar thickening system described in the present invention for use in hair colourants, bleaches or highlighting formulations .

SUMMARY OF THE INVENTION

The present invention relates to a hair colouring and hair bleaching composition comprising (i) at least one oxidizing agent, and (ii) at least one worm-like micelle thickening system comprising a) from 0.1 to 40.0 % of at least one ionic surfactant and b) at least one electrolyte as source of counter-ions for said ionic surfactant, wherein concentration C of said counter-ions is at least 0.25 mole/kg as defined according to the formula $C = \sum M*10^{(z-1)}$, wherein $\sum$ symbolizes summation, M is equal to the molar concentration of each counter ion and z is equal to the charge on each counter ion.

In further embodiment, the present invention relates to a hair colouring and or bleaching kit comprising i) an individually packaged first oxidizing component comprising at least one oxidizing agent and ii) an individually packaged second component comprising at least one worm-like micelle thickening system comprising a) from 0.1 to 40.0 % of at least one ionic surfactant and b) at least one electrolyte as source of counter-ions for said ionic surfactant, wherein concentration C of said counter-ions upon mixing of said first and second components i) and ii), is at least 0.25 mole/kg as defined herein.

A further embodiment of the present invention relates to a hair colouring or bleaching kit comprising i) an individually packaged first oxidising component comprising a) at least one source of hydrogen peroxide and b) from 0.1 to 40.0 % of at least one ionic surfactant and ii) an individually packaged second component comprising at least one electrolyte source of counter-ions for said ionic surfactant, wherein upon mixing said first and second components i) and ii), the resultant composition mixture has at least one worm-like micelle thickening system comprising said ionic surfactant and said counter-ions at a concentration C of at least 0.25 mole/kg as defined herein.

The present invention also relates to hair colouring and or bleaching kits comprising an individually packaged first component composition and an individually packaged second component composition, wherein said first and second component compositions independently have a viscosity of less than 1000cps, and wherein upon mixing said first and second component compositions, the resultant composition mixture has a viscosity of from 1000cps to 60000cps and wherein said resultant composition comprises a worm-like micelle phase thickening system and comprises less than 10% solvent.

The present invention also relates to the use of worm-like micelle phase thickening systems to thicken hair colouring and or hair bleaching and or hair highlighting compositions.

DETAILED DESCRIPTION OF THE INVENTION

While the specification concludes with claims, which particularly point out and distinctly claim the invention, it is believed the present invention will be better understood from the following description. As used herein the term "hair" to be treated may be "living" i.e. on a living body or may be "non-living" i.e. in a wig, hairpiece or other aggregation of non-living keratinous fibers. Mammalian, preferably human hair is preferred. However wool, fur and other keratin containing fibers are suitable substrates for the compositions according to the present invention.

All percentages are by weight of the total composition unless specifically stated otherwise. When more than one composition are used during a treatment, the total weight to be considered is the total weight of all the compositions applied on the hair simultaneously (i.e. the weight found "on head") unless otherwise specified. All ratios are weight ratios unless specifically stated otherwise. All molar concentrations are by weight of the total composition and presented as number of moles of component(s) in one kilogram of the composition, or "mole/kg".

The present invention relates to hair colouring and or bleaching and or highlighting compositions.

**Worm-like micelle phase thickening system**

According to the present invention, the hair colouring and or bleaching compositions comprise a worm-like micelle phase thickening system. The worm-like micelle thickening system of the present invention is defined as a thickening system comprising at least from 0.1 to 40.0% of at least one ionic surfactant and an electrolyte source of counter-ions for said ionic surfactant. The electrolyte source of counter ions has a total concentration C in the hair colouring and or bleaching composition defined according to formula:

$$C = \sum M*10^{(z-1)}$$

wherein M is equal to the molar concentration of each counter ion and z is equal to the charge on each counter ion.

According to the present invention the concentration of the electrolyte counter ion source is at least 0.25 mole/kg.

It has been surprisingly found that the wonn-like micelle thickening systems of the present invention can be utilized to provide a viscosity trigger for hair colouring and or bleaching systems. The system is particularly beneficial for application for so called thin-thin type liquid hair colouring or bleaching formulations, whereby upon upon mixing the two components the viscosity is increased to the desired level required for application. Moreover, the isotropic worm-like micelle systems usually have a clear or translucent appearance which may be desirable as it can connote cleanliness whilst also being readily opacified if a creamy appearance is desired. Furthermore, the systems of the present invention further allow for exceptional ease of mixing ensuring homogeneity for the consumer.

It has also been surprisingly found that the use of worm-like micelle thickening systems also allows for the elimination or significant reduction of the concentration of solvent typically required to incorporate dye precursors into hair colouring compositions and or provide the desired viscosity in oxidative hair colouring and or bleaching compositions. Those skilled in the art will recognize that high levels of solvents can be both detrimental to a system with respect to dye uptake, and in some cases detrimental with respect to mildness. Hence, the worm-like micelle thickening system of the present invention provides a useful route to increase the inherent mildness of a system.

The term worm-like micelle system is well known in the art and is used to refer to the isotropic micellar phase (L1) which is described in detail in Advances in Colloid and interface Science, 17 (1982) 275-298 and 26 (1986) 177-203, Journal of Physical Chemistry, Vol. 80, Number 9, April 22, 1976 pages 905-922, Journal of Physical Chemistry, 1988, 92, 4712-4719 and Langmuir 1992, 8, 2140-2146. All of these publications are incorporated herein by reference.

The term worm-like micelle phase thickening system as used herein refers to an isotropic micellar phase (L1-phase) wherein at least a portion, preferably at least 50%, more preferably substantially all of the ionic surfactant molecules are present in long cylindrical micelles (also know as worm or rod like micelles) whose rotational volumes overlap. Those skilled in the art will recognize that worm-like micelle phase thickening systems usually have a complex structure of entangled, elongated worm/rod shaped micelles. The rheology of these systems is highly influenced by the presence of electrolyte. The viscosity of the system is directly related to the length and associated degree of entanglement of the micelles which can be controlled by varying the surfactant concentration and by the addition of electrolytes. For a given surfactant concentration a viscosity maximum occurs with increasing electrolyte concentration beyond which additional electrolyte causes thinning of the system. The viscosity of the worm-like micelle phase thickening system of the present invention is at least 1000 cPs. Thus, the system can be formulated as a thin solution beyond the viscosity maximum with thickening occurring upon dilution.

Furthermore it has also been surprisingly found that the presence of oxidising agents also influences the thickening and thinning profiles of the aforementioned systems in that it requires specific high electrolyte concentration to achieve the desired viscosity.

The presence of worm-like micelle phases in solution can be readily identified by standard methods known in the art such as Cryo-Transition Electron Microscopy (TEM). The method is described in detail in Journal of Physical Chemistry, 1992, 96, 474-484 which is incorporated herein by reference and further described hereinbelow.

Methods of calculating the concentration of ions obtained from electrolytes are well known to those skilled in the art. The molar concentration M of each counter-ion of electrolytes formed by strong acids and strong bases is calculated according to the standard dissociation equations i.e. wherein the stoichiometric electrolyte formula is taken into consideration. For example, 58.5 grams of sodium chloride (NaCl, molecular weight 58.5) dissolved in 941.5 grams of water will produce 1 mole/kg of sodium cations ($Na^+$) and 1 mole/kg of chloride anions ($Cl^-$). In another example, 9.5 grams magnesium chloride ($MgCl_2$, molecular weight 95.2) dissolved in 990.5 grams of water will produce 0.1 mole/kg of magnesium cations ($Mg^{2+}$) and 0.2 moles/kg of chloride anions ($Cl^-$). In the latter example, the concentration C, according to the formula in the present invention, of chloride counter-ions with charge z=1 is 0.2 mole/kg, whereas the concentration C of magnesium counter-ions with charge z=2 is $C = \sum M*10^{(z-1)} = 0.1 *10^{(2-1)} = 0.1 *10 = 1.0$ mole/kg.

It should be noted that for electrolytes formed by a weak acid or a weak base or both, the counter-ions can participate in several equilibrium steps depending on the solution pH, producing ions with different charges (also producing non-charged species). Such equilibrium is commonly described with acid or base equilibrium constants, often represented on a logarithmic scale as $pK_a$ and $pK_b$ for acids and bases correspondingly. The values of these constants can be readily obtained from literature publications such as The Chapman and Hall Chemical Database. The solution pH is measured using the method described herein.

The concentration of counter-ions can be then calculated according to the procedure described below. For electrolytes containing ions of a weak acid at the total concentration $M_{total}$ (mole/kg), the concentration $M(H_nA)$ of the fully protonated form $H_nA$ where n is the number of hydrogen atoms is first calculated according to the equation (1)

$$M(H_nA) = M_{total} / [ 1 + 10^{-pKa1}/10^{-1*pH} + 10^{-pKa1} * 10^{-pKa2} /10^{-2*pH} + ... + 10^{-pKa1} * 10^{-pKa2} * ... * 10^{-pKan} /10^{-n*pH} ], \quad (1)$$

wherein $pK_a1$, $pK_a2$ ... $pK_an$ are the $pK_a$ of each individual dissociation steps. The concentration of each counter-ion containing (n-x) hydrogen atoms is then calculated according to the equation (2)

$$M(H_{n-x}A^{x-}) = M(H_nA) * [ 10^{-pKa1} * 10^{-pKa2} * ... * 10^{-pKax} /10^{-x*pH} ], \quad (2)$$

For electrolytes containing ions of a weak base, calculations similar to equations (1) and (2) can be performed, using values of $pK_b$ of corresponding bases ($pK_b$ =14 - $pK_a$) in place of $pK_a$ and values of pOH (pOH =14-pH) in place of pH.

It should be noted that species which do not possess positive or negative charges or possess both and equal positive and negative charges (zwitter-ionic) are not included into the calculation of the final counter-ion concentration C.

For example, 100 grams of 30% active ammonium hydroxide solution ($NH_4OH$, molecular weight 35.0) added to 900 grams of water at pH=10.0 (3% w/w solution) will produce the following concentration of positive counter-ions:

$$M_{total} = (100 * 30 / 100) / 35.0 = 0.86 \text{ mole/kg}$$

$$M(NH_4OH) = M_{total} / [ 1 + 10^{-pKb1}/10^{-1*pOH} ] = 0.86 / [ 1 + 10^{-4.75}/10^{-(14-10)} ] = 0.73 \text{ (mole/kg)}$$

$$M(NH_4^+) = M(NH_4OH) * [ 10^{-pKb1} /10^{-1*pOH} ] = 0.73 * [ 10^{-4.75}/10^{-(14-10)} ] = 0.13 \text{ (mole kg)}$$

($pK_{b1}$ of ammonium hydroxide is 4.75 as referred in The Chapman and Hall Chemical Database, density of solution is assumed equal to 1)

$NH_4OH$ is not charged and does not contribute to the total ion concentration. The concentration C of positive counter-ions ($NH_4^+$) with charge z=1 is therefore C = 0.13 mole/kg.

The following is based upon the formulation in Example 2 described hereinafter. The surfactant system in this example is anionic and therefore only positive ions need to be considered as counter-ions. The table below demonstrates the calculations at pH = 9.0:

| Ingredient | % w/ w | Cation | Cation conjugate base | MW | $pK_a$ / $pK_b$ | M mole/kg | z | C mole/kg |
|---|---|---|---|---|---|---|---|---|
| Ammonium Carbonate | 7.5 | $NH_a^+$ | Weak base | 97 | - / 4.75 | 0.99* | 1 | 0.99 |
| Sodium Glycinate | 5.0 | $Na^+$ | Strong base | 97 | n/a | 0.52 | 1 | 0.52 |
| EDTA (tetrasodium salt) | 0.1 | $Na^+$ | Strong base | 416 | n/a | 0.0096 | 1 | 0.0096 |
| Sodium sulphite | 0.1 | $Na^+$ | Strong base | 126 | n/a | 0.0079 | 1 | 0.0079 |

(continued)

| Ingredient | % w/ w | Cation | Cation conjugate base | MW | $pK_a$ / $pK_b$ | M mole/kg | z | C mole/kg |
|---|---|---|---|---|---|---|---|---|
| Total positive counter-ion concentration $C=\Sigma M*10^{(z-1)}$ | | | | | | | | **1.53** |
| $M(NH_4OH) = (2*75/97) / [1 + 10^{-4.75}/10^{-(14-9)}]$ * $= 0.56$ ; $M(NH_4^+) = 0.56 * 10^{-4.75}/10^{-(14-9)}$ $= 0.99$ (mole/kg) | | | | | | | | |

Further description of the methods to calculate ion concentrations can be found for example in H Rible "pH Buffer Theory - A New Approach", John Wiley & Sons, 1996.

## Ionic Surfactant

According to the present invention the worm-like micelle thickening system comprises an ionic surfactant. Suitable ionic surfactants for use herein may be selected from anionic surfactants, cationic surfactants and or mixtures thereof. Examples of anionic surfactants, which can be used, alone or as mixtures, for use herein include for example, salts of (for example, sodium salts, ammonium salts, amine salts, amino alcohol salts and magnesium salts) the following compounds: alkyl sulphates, alkyl ether sulphates, alkylamido ether sulphates, alkylarylpolyether sulphates, monoglyceride sulphates; alkyl sulphonates, alkyl glyceryl sulphonate, alkyl phosphates, alkylamide sulphonates, alkylaryl sulphonates, a-olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl ether phosphates; acyl sarcosinates; acyl isethionates, N-acyltaurates and mixtures thereof. The alkyl or acyl radical of all of these various compounds, for example, comprises from 8 to 24 carbon atoms, and the aryl radical, for example, is chosen from phenyl and benzyl groups. Among the anionic surfactants, which can also be used, mention may also be made of fatty acid salts such as the salts of lauric, myristic, oleic, ricinoleic, palmitic and stearic acids, coconut oil acid or hydrogenated coconut oil acid; acyl lactylates in which the acyl radical comprises from 8 to 20 carbon atoms. Weakly anionic surfactants can also be used, such as alkyl-D-galactosiduronic acids and their salts, as well as polyoxyalkylenated ($C_6$-$C_{24}$) alkyl ether carboxylic acids, polyoxyalkylenated ($C_6$-$C_{24}$) alkylaryl ether carboxylic acids, polyoxyalkylenated ($C_6$-$C_{24}$) alkylamido ether carboxylic acids and their salts, for example, those comprising from 2 to 50 ethylene oxide groups, and mixtures thereof. Anionic derivatives of polysaccharides, for example carboxyalkyl ether of alkyl polyglucosides, can be also used.

Preferably, the anionic surfactants are selected from those having an alkyl or acyl radical comprising from 8 to 16 carbon atoms and a Kraft point of less than 25°C.

Anionic surfactants particularly suitable for use herein include surfactants according to the formula $R_nX_mYM$, wherein R is a alkyl, alkenyl or alkylaryl group having from 8 to 30 carbon atoms and preferably from 8-18 carbon atoms, X is a polar group comprising at least one carbon atom and at least one oxygen or nitrogen atom, Y is an anionic group selected from sulphates, sulphonates, phosphates, phosphonates, and or carboxylates, n equals 1, m equals 1 or 2 and M is hydrogen or a salt forming cation and mixtures thereof.

Preferably, the anionic surfactants are selected from alkyl sulphates, alkyl phosphates, alkyl ether phosphates, alkyl ether sulphates, alkyl glyceryl sulphonates, N-acyl sarcosinates, N-acyl taurates, acyl lactylates and carboxyalkyl ether of alkyl polyglucosides and fatty acid salts and alkyl ether carboxylates and mixtures thereof.. Yet more preferably, the anioinc surfactants are selected from N-acyl sarcosinates, alkyl sulphates, alkyl phosphates, alkyl ether sulphates, alkyl ether phosphates with average 1 to 20, preferably 1-10 and most preferably 1-3 ethylene oxide units.

The anionic surfactants may also be preferably selected from those having an alkyl or acyl radical comprising 16 carbon atoms with on average 1.6 methyl branches per molecule or at least one unsaturated carbon bond.

The cationic surfactants suitable for use in the worm-like micelle thickening system of the present invention may be selected from quaternary ammonium salts or amido-amines having at least one fatty chain comprising from 8 to 30 carbon atoms and mixture thereof. Preferably, the cationic surfactants are selected from those having an alkyl or acyl radical comprising 8 to 18 carbon atoms and a Kraft point of less than 25°C.

Quaternary ammonium salts suitable for use herein as cationic surfactants have a general formula $N^+$

$(R_1R_2R_3R_4)$ $X^-$: wherein, $R_1$ is selected from linear and branched radicals comprising about 12 to 30 carbon atoms, $R_2$ is selected from linear and branched radicals comprising about 12 to 30 carbon atoms or the same group as radicals $R_3$ to $R_4$, the radicals $R_3$ to $R_4$, which may be identical or different, are selected from linear and branched aliphatic radicals comprising from about 1 to 4 carbon atoms, and aromatic radicals such as aryl and alkylaryl, the aliphatic radicals may comprise at least one hetero atom such as oxygen, nitrogen, sulphur and halogens, the aliphatic radicals are chosen, for example, from alkyl, alkoxy and alkylamide radicals, and wherein X- is an anion selected from halides such as chloride, bromide and iodide) (C2-C6)alkyl sulphates, such as methyl sulphate, phosphates, alkyl and alkylaryl sulphonates, and anions derived from organic acids, such as acetate and lactate.

The amido-amine suitable for use herein may be selected from compounds having the general formula $R'_1$-$CONH(CH_2)nNR'_2R'_3$: wherein, $R'_1$ is selected from linear and branched radicals comprising about 12 to 30 carbon atoms, the radicals $R'_2$ and $R'_3$, which may be identical or different, are selected from hydrogen, linear and branched aliphatic radicals comprising from about 1 to 4 carbon atoms, and aromatic radicals such as aryl and alkylaryl, the aliphatic radicals may comprise at least one hetero atom such as oxygen, nitrogen, sulphur and halogens, the aliphatic radicals are chosen, for example, from alkyl, alkoxy and alkylamide radicals, and wherein n is integer from 1 to 4.

Preferred cationic surfactants for use herein are cetyltrimethylammonium chloride, cetyltrimethylamidopropyldimethylamine, behenamidopropyldimethylamine and mixtures thereof.

According to the present invention the worm-like micelle thickening system may also comprise optional amphoteric or zwitterionic surfactants which can be selected, for example, from aliphatic secondary and tertiary amine derivatives in which the aliphatic radical is chosen from linear and branched chains comprising from 8 to 22 carbon atoms and comprising at least one water-soluble anionic group (for example carboxylate, sulphonate, sulphate, phosphate or phosphonate); mention may also be made of $(C_8$-$C_{20})$alkylbetaines, sulphobetaines, $(C_8$-$C_{20})$alkylamido$(C_1$-$C_6)$alkylbetaines, $(C_8$-$C_{20})$alkylamido$(C_1$-$C_6)$alkylsulphobetaines and mixtures thereof.

Among the amine derivatives, mention may be made of the products sold under the name Miranol, as described, for example, in U.S. Pat. Nos. 2,528,378 and 2,781,354 and having the structures of:

$$R_2\text{-CON }HCH_2CH_2\text{-}N^+(R_3)(R_4)(CH_2COO^-) \qquad \text{(VI)}$$

in which: $R_2$ is chosen from alkyl radicals derived from an acid $R_2$-COOH present in hydrolysed coconut oil, and heptyl, nonyl and undecyl radicals, $R_3$ is a β-hydroxyethyl group and $R_4$ is a carboxymethyl group; and of

$$R_5\text{-CONHCH}_2\text{CH}_2\text{-N(B)(C)} \qquad \text{(VII)}$$

wherein B represents $-CH_2CH_2OX'$, C represents $-(CH_2)_z-Y'$, with z=1 or 2, X' is chosen from the $-CH_2CH_2-COOH$ group and a hydrogen atom, Y' is chosen from -COOH and $-CH_2-CHOH-SO_3H$ radicals, $R_5$ is chosen from alkyl radicals of an acid $R_5$-COOH present in coconut oil or in hydrolysed linseed oil, alkyl radicals, such as $C_7$, $C_9$, $C_{11}$ and $C_{13}$ alkyl radicals, a $C_{17}$ alkyl radical and its iso form, and unsaturated $C_{17}$ radical. These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, and cocoamphodipropionic acid. Salts of diethyl aminopropyl cocoaspartamid can be also used.

Preferably the amphoteric surfactants are selected from those having an alkyl or acyl radical comprising 8 to 18 carbon atoms and a Kraft point of less than 25°C. Particularly preferable amphoteric surfactants for use here in are Cocamidopropyl betaine, sodium lauryl amphoacetate and mixtures thereof.

Particularly preferred worm-like micelle phase thickening systems according to the present invention include the combination of N-acyl sarcosinates, alkyl sulphates, alkyl phosphates, alkyl ether sulphates, alkyl ether phosphates with said ether phosphates having on average I to 20, preferably 1-10 and most preferably 1-3 ethylene oxide units and mixtures thereof and said amphoteric or zwitterionic surfactant is selected from cocoamidopropylbetaine, sodium lauryl amphoacetate and mixtures thereof. An alternatively preferred worm-like micelle phase thickening system includes the combination of an alkyl sulfate surfactant comprising from about 12 to 16 carbon atoms and an alkyl betaine having from 10 to 14 carbon atoms. Another particularly preferred worm-like micelle phase thickening systems according to the present invention includes the combination of alkyl sarcosinate comprising from about 12 to 18 carbon atoms and an alkyl betaine comprising from about 10 to 14 carbon atoms. Another particularly preferred example of the worm-like micelle thickening system comprises at least one branched alkyl sulfate comprising on average 18 carbon atoms, a branched alkyl ether sulfate comprising on average 18 carbons and on average 1-2 ethylene oxide units and an alkyl betaine comprising 10-14 carbon atoms.

More than one surfactant of the above specified types or any combination of the surfactants can be used in the present invention. The compositions of the present invention may comprise a total amount of worm-like micelle phase thickening system forming surfactants of from about 0.1% to about 40.0%, preferably from about 1% to about 15%, and

more preferably from about 2% to about 10% (by weight of the total composition).

The Kraft point of the surfactant(s) used according to the invention can be measured by a method described in Biochim. Biophysics, 1989, 988, 221-256.

## Electrolyte

According to the present invention the worm-like micelle phase thickening system comprises at least one electrolyte source of counter ions for the ionic surfactant. The term electrolyte as used herein refers to ionic salts or compounds that ionize in solution resulting in the presence of positive and negative ions. According to the present invention the term counter-ion as used herein refers to ions which are of opposite charge to that on the ionic surfactant used to form said system. For example, in an anionic surfactant based worm-like micelle thickening system, the counter ions are the cations.

Suitable electrolytes for use in the present invention result in the thickening of the ionic surfactant at a first concentration and thinning of the surfactant at a second higher concentration. Suitable electrolytes for use herein include alkaliser sources such as ammonium ion sources, carbonate ion sources, radical scavengers sources and mixtures thereof.

Suitable sources of alkalizer include sources of ammonium ions, for example ammonium chloride, ammonium sulphate, ammonium nitrate, ammonium phosphate, ammonium acetate and mixtures thereof.

Suitable electrolytes for use herein include but are not limited to sodium, potassium, lithium, calcium, magnesium, barium, ammonium salts of carbonate, carbamate, percarbonate, hydrogencarbonate ions, sulphate, phosphate, salicylate, chloride, bromide, iodide, fluoride and mixtures thereof such as ammonium carbonate, ammonium hydrogen carbonate, potassium carbonate, sodium carbonate, sodium hydrogen carbonate, potassium hydrogen carbonate, lithium carbonate, calcium carbonate, magnesium carbonate, barium carbonate, sodium chloride, magnesium chloride, potassium chloride, sodium sulphate, potassium sulphate, magnesium sulphate, ammonium sulphate, sodium salicylate, potassium salicylate and mixtures thereof.

Another preferred type of electrolyte is a radical scavenger source as defined hereinafter. Example of such radical scavengers include for example potassium, sodium and ammonium salts of glycine, sarcosine, lysine, serine, glutamic acid and mixtures thereof.

In a particularly preferred embodiment of the present invention, the electrolyte source is selected from ammonium carbonate, ammonium hydrogen carbonate, ammonium carbamate, sodium glycinate or mixtures thereof.

Preferably, the electrolyte will react with the oxidant to form additional oxidizing species. More preferably, the electrolyte will react with the oxidant to form peroxymonocarbonate ions. Examples of such electrolytes include ammonium carbonate, ammonium hydrogencarbonate, ammonium carbamate, potassium carbonate, sodium carbonate, sodium hydrogen carbonate, potassium carbonate, potassium hydrogen carbonate and mixtures thereof.

Sources of electrolyte may alternatively be provided from other components in the hair colourant and or bleach compositions such as surfactants, dyes, preservatives, antioxidants and residual salt present from any components in the composition.

The total amount of electrolyte in the composition of the present invention are such that the compositions have at least one electrolyte as source of counter-ions for said ionic surfactant wherein the concentration C of said counter-ions defined according to formula $C = \sum M*10^{(z-1)}$ is at least 0.25 mole/kg , preferably from 0.5mole/kg to 4.0mole/kg, more preferably from 1.0mole/kg to 3.0mole/kg; wherein M is equal to the molar concentration of each counter ion and z is equal to the charge on corresponding counter ion.

## Oxidizing agent

The compositions according to the present invention comprise at least one source of an oxidizing agent. Preferred oxidizing agents for use herein are water-soluble peroxygen oxidizing agents. "Water-soluble" as defined herein means that in standard condition at least 0.1g, preferably 1g, more preferably 10g of said oxidizing agent can be dissolved in 1 liter of deionized water. The oxidizing agents are valuable for the initial solubilisation and decolourisation of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is

dissolved prior to use. Preferred for use in the compositions according to the present invention are hydrogen peroxide, percarbonate, persulphates and combinations thereof.

According to the present invention the compositions comprise from about 0.1% to about 15% by weight, preferably from about 1% to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably from 7.5 to 9.5 more preferably from 8.4 to 9.5 and most preferably at about pH 9. Moreover, this system is also particularly effective in combination with a source of ammonia or ammonium ions. It has been found that this oxidizing agent can deliver improvements to the desired hair colour results particularly with regard to the delivery of high lift, whilst considerably reducing the odour, skin and scalp irritation and damage to the hair fibres.

Accordingly, any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the compositions comprise from about 0.1 % to about 15% by weight, preferably from about 1% to about 10% by weight, and most preferably from about 1% to about 8% by weight of a hydrogencarbonate ion and from about 0.1% to about 10% by weight, preferably from about 1% to about 7% by weight, and most preferably from about 2% to about 5% by weight of a source of hydrogen peroxide.

**Additional components**

The compositions of the present invention may further comprise additional ingredients which include, but are not limited to; alkalising agents, additional surfactants, hair dyeing agents such as oxidative dye precursors, non-oxidative pre-formed dyes, additional thickeners and /or rheology modifiers, opacifiers such as mica, solvents, enzymes, conditioning agents, carriers, antioxidants, stabilizers, chelants, perming actives, perfume, reducing agents, hair swelling agents and/or polymers. Some of these additional components are detailed hereafter.

**Source of alkalizing agent**

According to the present invention the composition may optionally comprise at least one source of alkalizing agent, preferably a source of ammonium ions and or ammonia. Particularly, preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium hydrogen carbonate, ammonium carbamate, ammonia and mixtures thereof. The compositions of the present invention may comprise from about 0.1% to about 10% by weight, preferably from about 0.5% to about 5%, most preferably from about 1% to about 3% of an alkalizing agent, preferably ammonium ions. Preferably, if present, the ammonium ions and carbonate ions are present in the composition at a weight ratio of from 3:1 to 1:10, preferably 2:1 to 1:5.

Preferably, the compositions of the present invention have a pH of from about 11 to about 7.5, more preferably from about 9.5 to about 8.4 and most preferably from about 9.4 to about 8.5 and even more preferably about pH 9.0.

The pH of the compositions can be determined by using either a Mettler Toledo MP220 or a MP225 pH equipment, fitted with a standard laboratory pH electrode. The equipment is calibrated before each use using standard calibration buffers and using standard calibration procedure.

**Surfactants**

The compositions according to the present invention may further comprise at least about 0.01% of one or more additional surfactants to those utilised in the worm-like micelle phase thickening system of the present invention. It is believed that these additional surfactant(s) may or may not reside fully or partially in the micelles however they typically do not affect the viscosity of the thickening system. Surfactants suitable for use herein generally have a lipophilic chain length of from about 8 to about 30 carbon atoms and can be selected from anionic, nonionic, amphoteric and cationic

surfactants and mixtures thereof. Particularly preferred are amphoteric surfactants and mixtures thereof.

The nonionic surfactants are compounds that are well known (see, for example, in this respect "Handbook of Surfactants" by M. R. Porter, published by Blackie & Son (Glasgow and London), 1991, pp. 116-178). They can be selected , for example, from polyethoxylated, polypropoxylated and polyglycerolated fatty acids, alkyl phenols, $\alpha$-diols and alcohols comprising a fatty chain comprising, for example, from 8 to 18 carbon atoms, it being possible for the number of ethylene oxide or propylene oxide groups to range, for example, from 2 to 200 and for the number of glycerol groups to range, for example, from 2 to 30. Mention may also be made of copolymers of ethylene oxide and of propylene oxide, condensates of ethylene oxide and of propylene oxide with fatty alcohols; polyethoxylated fatty amides preferably having from 2 to 30 mol of ethylene oxide and their momoethanolamine and diethanolamine derivatives, polyglycerolated fatty amides, for example, comprising on average from 1 to 5, and such as from 1.5 to 4, glycerol groups; polyethoxylated fatty amines such as those containing from 2 to 30 mol of ethylene oxide; oxyethylenated fatty acid esters of sorbitan having from 2 to 30 mol of ethylene oxide; fatty acid esters of sucrose, fatty acid esters of polyethylene glycol, alkylpolyglycosides, N-alkylglucamine derivatives, amine oxides such as ($C_{10}$-$C_{14}$)alkylamine oxides or N-acylaminopropylmorpholine oxides.

## Hair dyes

The hair compositions of the present invention are preferably hair colouring compositions which comprise but are not limited to oxidative dyeing compositions. Such compositions comprise oxidative hair dye precursors also known as primary intermediates and couplers that will deliver a variety of hair colors to the hair. These small molecules are activated by the oxidizing agent and react with further molecules to form a larger colored complex in the hair shaft.

The precursors can be used alone or in combination with other precursors, and one or more can be used in combination with one or more couplers. Couplers (also known as color modifiers or secondary intermediates) are generally colorless molecules that can form colors in the presence of activated precursors, and are used with other precursors or couplers to generate specific color effects or to stabilize the color. The choice of precursors and couplers will be determined by the color, shade and intensity of coloration that is desired. The precursors and couplers can be used herein, singly or in combination, to provide dyes having a variety of shades ranging from ash blonde to black.

These compounds are well known in the art, and include aromatic diamines, aminophenols, aromaticdiols and their derivatives (a representative but not exhaustive list of oxidation dye precursor can be found in Sagarin, "Cosmetic Science and Technology", "Interscience, Special Edn. Vol. 2 pages 308 to 310). It is to be understood that the precursors detailed below are only by way of example and are not intended to limit the compositions and processes herein. These are:

1,7-Dihydroxynaphthalene (1,7-NAPHTHALENEDIOL), 1,3-Diaminobenzene ( m-PHENYLENEDIAMINE), 1-Methyl-2,5-diaminobenzene (TOLUENE-2,5-DIAMINE), 1,4-Diaminobenzene (p-PHENYLENEDIAMINE), 1,3-Dihydroxybenzene (RESORCINOL), 1,3-Dihydroxy-4-chlorobenzene, (4-CHLORORESORCINOL), 1-Hydroxy-2-aminobenzene, (o-AMINOPHENOL), 1-Hydroxy-3-aminobenzene (m-AMINOPHENOL), 1-Hydroxy-4-amino-benzene (p-AMINOPHENOL), 1-Hydroxynaphthalene (1-NAPHTHOL), 1,5-Dihydroxynaphthalene (1,5-NAPHTHALENEDIOL), 2,7-dihydroxynaphthalene (2,7-NAPHTHELENEDIOL) 1-Hydroxy-2,4-diaminobenzene (4-DIAMINOPHENOL), 1,4-Dihydroxybenzene (HYDROQU1NONE), 1-Hydroxy-4-methylaminobenzene (p-METHYLAMINOPHENOL), 6-Hydroxybenzo-morpholine (HYDROXYBENZOMORPHOLINE), 1-Methyl-2-hydroxy-4-aminobenzene (4-AMINO-2-HYDROXY-TOLUENE), 3,4-Diaminobenzoic acid (3,4-DIAMINOBENZOIC ACID), 1-Methyl-2-hydroxy-4-(2'-hydroxyethyl)aminobenzene (2-METHYL-5-HYDROXYETHYLAMINO-PHENOL), 1,2,4-Trihydroxybenzene (1,2,4-TRIHYDROXYBENZENE), 1-Phenol-3-methylpyrazol-5-on (PHENYLMETHYLPYRAZOLONE), 1-(2'-Hydroxyethyloxy)-2,4-diaminobenzene (2,4-DIAMINOPHENOXY-ETHANOL HCL), 1-Hydroxy-3-amino-2,4-dichlorobenzene (3-AMINO-2,4-DICHLORO-PHENOL), 1,3-Dihydroxy-2-methylbenzene (2-METHYLRESORCINOL), 1-Amino-4-bis-(2'-hydroxyethyl)aminobenzene (NN-BIS(2-HYDROXY-ETHYL)-p-PHENYLENEDIAMINE), 2,4,5,6-Tetraaminopyrimidine (HC Red 16), 1-Hydroxy-3-methyl-4-aminobenzene (4-AMINO-m-CRESOL), 1-Hydroxy-2-amino-5-methylbenzene (6-AMINO-m-CRESOL), 1,3-Bis-(2,4-Diaminophenoxy)propane (1,3-BIS-(2,4-DIAMINOPHENOXY)-PROPANE),1-(2'-Hydroxyethyl)-2,5-diaminobenzene (HYDROXYETHYL-p-PHENYLENE DIAMINE SULPHATE), 1-Methoxy-2-amino-4-(2'-hydroxyethylamino)benzene, (2-AMINO-4-HYDROXYETHYLAMINOANISOLE) 1-Hydroxy-2-methyl-5-amino-6-chlorobenzene (5-AMINO-6-CHLORO-o-CRESOL), 1-Hydroxy-2-amino-6-methylbenzene (6-AMINO-o-CRESOL), 1-(2'-Hydroxyethyl)-amino-3,4-methylenedioxybenzene (HYDROXYETHYL-3,4-METHYLENEDIOXY-ANILINE HCl), 2,6-Dihydroxy-3,4-dimethylpyridine (2,6-DIHYDROXY-3,4-DIMETHYLPYRIDINE), 3,5-Diamino-2,6-dimethoxypyridine (2,6-DIMETHOXY-3,5-PYRIDINEDIAMINE), 5,6-Dihydroxyindole (,DIHYDROXY-INDOLE), 4-Amino-2-aminomethylphenol (2-AMINOETHYL-p-AMINO-PHENOL HCl), 2,4-Diamino-5-methylphenetol (2,4-DIAMINO-5-METHYL-PHENETOLE HCl), 2,4-Diamino-5-(2'-hydroxyethyloxy)toluene (2,4-DIAMINO-5-METHYLPHENOXYETHANOL HCl), 5-Amino-4-chloro-2-methylphenol (5-AMINO-4-CHLORO-o-CRESOL), 4-Amino-1-hydroxy-2-(2'-hydroxyethylaminomethyl)benzene HYDROXYETHYLAMINOMETHYL-p-AMINO PHENOL HCl), 4-Amino-1-hydroxy-2-methoxymethylbenzene (2-METHOXYMETHYL-p-AMINOPHENOL HCl), 1,3-Bis(N(2-Hydroxyethyl)N(4-aminophenyl)amino)-2-propanol (HYDROXYPROPYL-BIS-(N-HYDROXY-ETHYL-p-PHENYLENEDIAMINE)HCL), 6-Hydorxyindole (6-HYDROXY-INDOLE), 2,3-Indolinedione (ISATIN), 3-Amino-2-methylamino-6-methoxypyridine (HC BLUE NO. 7), 1-Phenyl-3-methyl-5-pyrazolone-2,4-dihydro-5,2-phenyl-3H-pyrazole-3-one, 2-Amino-3-hydroxypyridine (2-AMINO-3-HYDROXYPYRIDINE), 5-Amino-salicylic acid, 1 -Methyl-2,6-bis(2-hydroxy-ethylamino)benzene (2,6-HYDROXYETHYLAMINO-TOLUENE), 4-Hydroxy-2,5,6-triaminopyrimidine (2,5,6-TRIAMINO-4-PYRIMIDINOL SULPHATE), 2,2'-[1,2-Ethanediyl-bis-(oxy-2,1-ethanediyloxy)]-bis-benzene-1,4-diamine (PEG-3,2',2'-DIp-PHENYLENEDIAMINE), 5,6-Dihydroxyindoline (DIHYDROXYINDOLYNE), N,N-Dimethyl-3-ureidoaniline (m-DIMETHYL-AMINO-PHENYLUREA), 2,4-Diamino-5-fluortoluenesulfatehydrate (4-FLUORO-6-METHYL-m-PHENYLENEDIAMTNE SULPHATE) and 1-Acetoxy-2-methylnaphthalene (1-HYDROXYYETHYL-4,5-DIAMINOPYRAZOLE SULPHATE). These can be used in the molecular form or in the form of peroxide-compatible salts.

The hair colouring compositions of the present invention may also include non oxidative hair dyes. i.e. direct dyes which may be used alone or in combination with the above described oxidative dyes. Suitable direct dyes include azo or anthraquinone dyes and nitro derivatives of the benzene series and or melanin precursors and mixtures thereof. Such direct dyes are particularly useful to deliver shade modification or highlights. Particularly preferred are Basic Red 51, Basic Orange 31, Basic Yellow 87 and mixtures thereof.

The hair dye compositions of the present invention will generally comprise from about 0.001% to about 10% of dyes. For example compositions providing low intensity dyeing such as natural blonde to light brown hair shades generally comprise from about 0.001% to about 5%, preferably from about 0.1 % to about 2%, more preferably from about 0.2% to about 1% by weight of dyeing composition of precursors and couplers. Darker shades such as browns and black typically comprise from 0.001% to about 10% by weight, preferably from about 0.05% to about 7% by weight, more preferably from about 1% to about 5% of precursors and couplers.

**Polymers**

The composition of the present invention may optionally further comprise at least about 0.01% of polymer as an additional thickener, rheology modifier, stabilizer and/or conditioning agent as described below.

The polymer can be chosen, for example, from associative polymers. As used herein, the expression "associative polymer" means an amphiphilic polymer comprising both hydrophilic units and hydrophobic units, for example, at least one C8-C30 fatty chain and at least one hydrophilic unit. Representative associative polymers that may be used are associative polymers chosen from:

(i) nonionic amphiphilic polymers comprising at least one fatty chain and at least one hydrophilic unit; for example celluloses or hydroxyethylcelluloses modified with groups comprising at least one fatty chain, hydroxypropyl guars modified with groups comprising at least one fatty chain, polyether urethanes comprising at least one fatty chain, copolymers of vinylpyrrolidone and of fatty-chain hydrophobic monomers, copolymers of hydrophilic acrylates or methacrylates and of hydrophobic monomers comprising at least one fatty chain.

(ii) anionic amphiphilic polymers comprising at least one hydrophilic unit and at least one fatty-chain unit; these, for example, may be chosen from those comprising at least one fatty-chain allyl ether unit and at least one hydrophilic unit comprising an ethylenic unsaturated anionic monomeric unit, or from those comprising at least one hydrophilic unit of unsaturated olefinic carboxylic acid type, and at least one hydrophobic unit of the type such as a (C8-C30) alkyl ester or oxylakylenated (C8-C30) alkyl ester of an unsaturated carboxylic acid; anionic amphopilic polymers may be further cross-linked.

(iii) cationic amphiphilic polymers comprising at least one hydrophilic unit and at least one fatty-chain unit; these, for example, may be chosen from quaternized cellulose derivatives and polyacrylates comprising amino side groups.

(iv) amphoteric amphiphilic polymers comprising at least one hydrophilic unit and at least one fatty-chain unit; mention may be made, for example, of methacrylamidopropyltrimethylammonium chloride/acrylic acid/C10-C30 alkyl methacrylate copolymers, wherein the alkyl radical is, for example, a stearyl radical.

Further, the polymer can be chosen from crosslinked acrylic acid homopolymers, crosslinked copolymers of (meth)acrylic acid and of (C1-C6)alkyl acrylate or polysaccharides. The polymer may also serve as conditioning agents, as described below.

Particularly preferred are acrylates/stereath-20 methacrylate copolymer (Aculyn 22 supplied by Rohm and Haas) and acrylates copolymer (Aculyn 33 supplied by Rohm and Haas) and Xanthan gum, Carbopol, Rheozan and mixtures thereof. The polymer will generally be used at levels of from about 0.01% to about 20.0% by weight of the composition, preferably of from about 0.1% to about 5%.

## Conditioning agent

The compositions of the present invention may comprise or are used in combination with a composition comprising a conditioning agent. Conditioning agents suitable for use herein are selected from silicone materials, amino silicones, fatty alcohols, polymeric resins, polyol carboxylic acid esters, cationic polymers, cationic surfactants, insoluble oils and oil derived materials and mixtures thereof. Additional materials include mineral oils and other oils such as glycerin and sorbitol.

The conditioning agent will generally be used at levels of from about 0.05% to about 20% by weight of the composition, preferably of from about 0.1% to about 15%, more preferably of from about 0.2% to about 10%, even more preferably of from about 0.2% to about 2%.

Particularly useful conditioning materials are cationic polymers and silicones. Conditioners of cationic polymer type may be chosen from those already known by those skilled in the art as improving at least one cosmetic properties of keratin fibres treated with a cosmetic composition. Cationic polymers can be chosen from those comprising units of at least one amine group chosen from primary, secondary, tertiary and quaternary amine groups that may either form part of the main polymer chain, or be borne by a side substituent that is directly attached to the main polymer chain.

Silicones can be selected from polyalkylsilioxane oils, linear polydimethylsiloxane oils containing trimethylsilyl or hydroxydimethylsiloxane endgroups, polymethylphenylsiloxane polydimethylphenylsiloxane or polydimethyldiphenylsiloxane oils, silicone resins, organofunctional siloxanes having in their general structure one or a number of organofunctional group(s), the same or different, attached directly to the siloxane chain. Said organofunctional group(s) are selected from: polyethylencoxy and / or polypropyleneoxy groups, (per)fluorinated groups, thiol groups, substituted or unsubstituted amino groups, carboxylate groups, hydroxylated groups, alkoxylated groups, quaternium ammonium groups, amphoteric and betaine groups. The silicone can either be used as a neat fluid or in the form of an pre-formed emulsion. Particularly preferred are amino silicones.

## Chelants

According to the present invention the compositions may comprise chelants. Chelants are well known in the art and refer to a molecule or a mixture of different molecules each capable of forming a chelate with a metal ion. Chelants are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996) both incorporated herein by reference.

Examples of chelants suitable for use herein include EDDS (ethylenediaminedisuccinic acid), carboxylic acids (in particular aminocarboxylic acids), phosphonic acids (in particular aminophosphonic acids) and polyphosphoric acids (in particular linear polyphosphoric acids), their salts and derivatives.

Chelants may be incorporated into the composition of the present invention as stabilizers and or preservatives. In addition it has also been found that chelants provide hair fibre damage benefits and thus they may be utilized in order to further improve the hair damage profile of the present invention. Levels of chelants in the present invention may be as low as about 0.1%, preferably at least about about 0.25%, more preferably about 0.5% for the most effective chelants such as diamine-N,N'-dipolyacid and monoamine monoamide-N,N'-dipolyacid chelants (for example EDDS). Less effective chelants will be more preferably used at levels of at least about 1%, even more preferably above about 2% by weight of the composition, depending of the efficiency of the chelant. Levels as high as about 10% can be used, but above this level significant formulation issues may arise. The majority of chelants will provide a source of electrolyte counter ion to the ionic surfactant as discussed hereinabove.

## Solvents

The compositions of the present invention optionally comprise a solvent system, in addition to water. As used herein, the term solvent system refers to a solvent system comprising all the solvents in the composition with the exception of water.

Suitable solvents for use in the solvent system herein include, but are not limited to, amides, esters, ethers, ketones, cyclic amides, cyclic esters, cyclic ketones, cyclic ethers, and mixtures thereof Nonlimiting examples of such solvents include ethyl formate, dimethyl isosorbide, acetylacetone, 2-butanone, acetone, methyl acetate, ethyl acetate, propyl acetate, ethoxyethanol, dipropylene glycol monomethyl ether, butyl lactate, t-butyl alcohol, phenyl acetate, 2-propoxyethanol, isopropoxyethanol, methoxypropanol, isopropyl lactate, hexyl alcohol, butoxyethanol, tripropylene glycol (PPG-3), triacetin, methoxyethanol, isopropyl alcohol, PEG-8, methyl lactate, PEG-6, PEG-5, PEG-4, N-methylpyrrolidone, propyl alcohol, dipropylene glycol (PPG-2), acetonitrile, phenoxyethanol, triethylene glycol, hexylene glycol, ethyl alcohol, γ-butyrolactone, butylene glycol, propylene carbonate, dimethyl sulfoxide, diethylene glycol, ethoxydiglycol, propylene glycol, pyrrolidone, pyrrolidone-2, methyl alcohol, ethylene carbonate, ethylene glycol, acetamide, glycerin,

butyl carbitol, 1,3-dioxolane, dimethoxymethane, 1,2-hexanediol, dipropylene glycol butyl ether, dipropylene glycol t-butyl ether, propionaldehyde, diethoxymethane and glycerol formal.

Preferred solvents for use in the solvent system herein include lower alkanols, C2-C6 polyols, glycol mono-lower alkyl ethers, diglycol mono-lower-alkyl ethers, and N-lower alkylpyrrolidones. The term "lower" refers to the number of carbon atoms being 3 or less. Specific examples include lower alcanols such as Ethanol, Isopropyl alcohol, lower polyols such as ethylene glycol, propyleneglycol, 1,3-butanediol, diethyleneglycol, glycerine; glycol monoethers such as 2-methoxyethanol and 2-ethoxyethanol; diglycol mono-lower alkyl ethers such as methoxydiglycol, ethoxydiglycol and N-lower-alkylpyrrolidones such as N-methylpyrrolidone and N-ethylpyrrolidone.

According to the present invention due to the presence of the worm-like micelle phase thickening system the hair colouring and or bleaching compositions require less solvent that in typical formulations and thus comprise less than 10%, preferably less than 8% more preferably less than 5% solvent.

**Radical Scavenger**

The hair colouring and bleaching composition of the present invention may comprise a source of radical scavenger which may be used as an electrolyte for the worm-like micelle phase thickening system of the present invention. As used herein the term radical scavenger refers to a species that can react with a radical, preferably carbonate radical to convert the radical by a series of fast reactions to a less reactive species.

Suitable radical scavengers for use herein include compounds according to the general formula:

(I): $R^1\text{-}Y\text{-}C(H)(R^3)\text{-}R^4\text{-}(C(H)(R^5)\text{-}Y\text{-}R^6)_n$

wherein Y is $NR^2$, O, or S, preferably $NR^2$, n is 0 to 2, and wherein $R^4$ is monovalent or divalent and is selected from: (a) substituted or unsubstituted, straight or branched, alkyl, mono- or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein $R^4$ can be connected to $R^3$ or $R^5$ to create a 5, 6 or 7 membered ring; and wherein $R^1$, $R^2$, $R^3$, $R^5$, and $R^6$ are monovalent and are selected independently from: (a), (b) and (c) described herein above, or H.

Preferably, $R^4$ is selected from: (a) substituted or unsubstituted, straight or branched, alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; more preferably $R^4$ is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.

Preferably, the $R^4$ systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; and most preferred is O.

Preferably, $R^1$, $R^2$, $R^3$, $R^5$, and $R^6$ are selected independently from any of the systems defined for $R^4$ above, and H. In alternative embodiments, any of $R^1$, $R^2$, $R^3$, $R^4$, $R^5$, and $R^6$ groups are substituted. Preferably, the substituent (s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, mono-, poly-, or per-fluoro alkyl systems; said systems of (i), (ii) and (iii) comprising from 1 to 10 carbon atoms and 0 to 5 heteroatoms selected from O, S, N, P, and Si; (b) the group of S-linked monovalent substituents consisting of $SA^1$, SCN, $SO_2A^1$, $SO_3A^1$, $SSA^1$, SOA', $SO_2NA^1A^2$, $SNA^1A^2$, and $SONA^1A^2$; (c) the group of O-linked monovalent substituents consisting of $OA^1$, OCN and $ONA^1A^2$; (d) the group of N-linked monovalent substituents consisting of $NA^1A^2$, $(NA^1A^2A^3)^+$, NC, $NA^1OA^2$, $NA^1SA^2$, NCO, NCS, $NO_2$, N=NA', N=NOA', $NA^1CN$, $NA^1NA^2A^3$; (e) the group of monovalent substituents consisting of $COOA^1$, $CON_3$, $CONA^1_2$, $CONA^1COA^2$, $C(=NA^1)NA^1A^2$, CHO, CHS, CN, NC, and X; and (f) the group consisting fluoroalkyl monovalent substituents consisting of mono-, poly-, or per-fluoro alkyl systems comprising from 1 to 12 carbon atoms and 0 to 4 heteroatoms.

For the groups (b) to (e), described above, $A^1$, $A^2$, and $A^3$ are monovalent and are independently selected from: (I) H, (2) substituted or unsubstituted, straight or branched, alkyl, mono- or poly-unsaturated alkyl, heteroalkyl, aliphatic, heteroaliphatic, or heteroolefinic systems, (3) substituted or unsubstituted, mono- or poly-cyclic aliphatic, aryl, or heterocyclic systems, or (4) substituted or unsubstituted, mono-, poly-, or per-fluoro alkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and I.

Preferred substituents for use herein include those having a Hammett Sigma Para ($\sigma_p$) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375).

Alternative suitable radical scavengers for use herein are compounds according to the general formula (II):

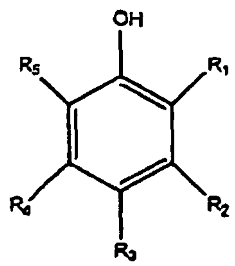

(II)

wherein $R_1$, $R_2$, $R_3$, $R_4$, and $R_5$ are each independently selected from H, $COO^-M^+$, Cl, Br, $SO_3^-M^+$, $NO_2$, $OCH_3$, OH or a $C^1$ to $C^{10}$ primary or secondary alkyl and M is either H or alkali metal. Preferably, the above-described radical scavengers have a pKa of more than 8.5 to ensure protonation of the hydroxy goup.

Other suitable radical scavengers for use herein include those selected from group (III) benzylamine, imidazole, di-tert-butylhydroxytoluene, hydroquinone, guanine, pyrazine, piperidine, morpholine, methylmorpholine, 2methyoxyethylamine, and mixtures thereof

Preferred radical scavengers according to the present invention are selected from the classes of alkanolamines, amino sugars, amino acids, esters of amino acids and mixtures thereof. Particularly preferred compounds are: monvethanolamine, 3-amino-1-propanol, 4-amino-1-butanol,5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof.

The radical scavengers according to the present invention preferably have a molecular weight of less than about 500, preferably less than about 300, more preferably less than about 250 in order to facilitate penetration of the radical scavenger into the hair fibre. The compositions of the present invention preferably comprise from about 0.1% to about 10% by weight, preferably from about 1% to about 7% by weight of radical scavenger. The radical scavenger is also preferably selected such that it is not an identical species as the alkalizing agent. According to one embodiment of the present invention the radical scavenger may be formed insitu in the hair dyeing compositions prior to application to the hair fibres.

**Method of Use**

It is understood that the examples of methods of use and embodiments described herein are for illustrative purposes only and that various modifications or changes in light thereof will be suggested to one skilled in the art without departing from the scope of the present invention.

Oxidative hair colouring compositions are usually sold in kits comprising, in individually packaged components such as separate containers, a dye component (also called "dye cream" for emulsions or "dye liquid" for solutions) comprising the oxidative dye, precursors and alkalizing agent which is typically ammonia in a suitable carrier and; a hydrogen peroxide component (also called "hydrogen peroxide cream" for emulsions or "hydrogen peroxide liquid" for solutions) comprising the oxidizing agent (usually hydrogen peroxide). The consumer mixes the dye component and hydrogen peroxide component together immediately before use and applies it onto the hair.

Similarly, bleaching compositions are also usually sold as a kit comprising two or three individually packaged components typically in two or three separate containers. The first component comprises the ammonium ion source (e.g. ammonia), the second component comprises the oxidizing agent and the third (optional) component comprises a second oxidizing agent. The bleaching compositions are obtained by mixing the above-mentioned compositions immediately before use and application to the hair.

After working the mixture for a few minutes (to insure uniform application to all of the hair), the oxidative dye or bleach composition is allowed to remain on the hair for an amount sufficient for the dyeing or bleaching to take place

(usually from about 2 to 60 minutes, typically about 30 to 45 minutes). The consumer then rinses his/her hair thoroughly with water and allows it to dry. It is observed that the hair has changed from its original color to the desired color.

When present in the oxidative dye compositions and bleaching compositions, the optional conditioning agent can be provided in a third container. In the latter case, all three compositions can be mixed immediately before use and applied together, or the content of the third container can be applied (after an optional rinse step) as a post-treatment immediately after the oxidative dye composition or bleaching composition resulting from the mixture of the other containers.

For the oxidative hair dye compositions the worm-like micelle phase thickening system may be comprised within the dye component or the hydrogen peroxide component or in both components. Alternatively the worm-like micelle phase thickening system may have individual components thereof distributed between the various components of the hair colouring or bleaching kits such that the worm-like micelle phase thickening system is formed upon mixing of the individual components.

The resultant mixed hair colouring or bleaching compositions according to the present invention thus preferably have a viscosity of from 1000 to 60000 cPs, preferably from 2000 to 30000 cPs and most preferably from 3000 to 25000 cPs. Moreover, prior to mixing the hair dye component (second component) and or the oxidizing component (first component) may have viscosity of less than 1000 cPs; such compositions are often referred as "thin-thin" or "liquid" colorant. The viscosity of the resultant mixture of first oxidative and second components i) and ii) in other words the hair colouring or bleaching composition that is applied on head is from 1000 to 60000 cPs, preferably from 2000 to 30000, more preferably form 3000 to 25000 cPs.

In another embodiment of the present invention the oxidative hair dye or bleaching compositions may comprise as an optional fourth component a colour referesher composition. Such colour refresher compositions comprise at least one pre-formed dye and may be applied to the hair immediately after the oxidative colour i.e. from about 1 minute after oxidative hair dye or bleach application to 60 days after the application. These colour refresher composition can be used to increase the intial colour obtained and or boost the colour during the wash and style cycle until the next oxidative colouring or bleaching event.

In yet another embodiment of the present invention the hair colouring and or bleaching and or highlighting compositions further comprises a device for the application of the composition onto the hair of the consumer. Such devices are known in the art and include, brushes and combs, which may be directly attached to the composition container (s) or used separately, and highlighting caps and foils and the like.

According to the present invention the method of colouring or bleaching hair also comprise embodiments whereby the composition is applied to the hair and preferably the mixture is worked for a few minutes (to insure uniform application to all of the hair). The composition is then allowed to remain on the hair in order for the colour to develop for a time period of less than about 20 minutes, preferably less than about 15 minutes, more preferably from about 5 minutes to about 10 minutes, most preferably for about 10 minutes. The consumer then rinses his/her hair thoroughly with water and allows it to dry and or styles the hair as usual. Such method provides additional convenience to consumer by permitting faster colouring or bleaching process.

According to an alternative embodiment of the present invention, the method of colouring and or bleaching the hair is also a sequential oxidative hair colouring or hair bleaching method comprising the steps of at least two sequential oxidative hair colour or hair bleaching treatments wherein the time period between each treatment is from 1 to 60 days, preferably from I to 40 days, more preferably from I to 28 days, even more preferably from I to 14 days and most preferably from 1 to 7 days. In such embodiments the time that the composition is retained on head may be less than about 20 minutes and is preferably less than about 10 minutes and most preferably from about 2 minutes to about 5 minutes. This method allows consumer to perform colouring or bleaching process in a way similar to conventional hair washing or conditioning process.

The kits described hereinabove are well known in the art and the composition in each container can be manufactured utilizing any one of the standard approaches, these include for example hot or cold mixing processes. For example, when using "cold mixing" process, surfactants and electrolyte of the present invention are added to approximately 50% of total water amount of the composition at ambient temperature, and mixed for 30 to 60 min, thus forming worm-like micelle thickening premix; this premix is then mixed cold with remaining amounts of water and other optional components thus forming first part of the above described bleaching or colouring kit. Second part of the bleaching and coloring kit can be manufactured by preheating water to 80°C, adding all high melting point optional components, and then cooling to less than 40°C adding oxidising agent and the remaining optional components and homogenising for 30 to 60 mins.

## Test Methods

### Worm-like micelle phase test method

The presence of worm-like micelle phases in solution can be readily identified by standard methods known in the art such as Cryo-Transition Electron Microscopy (TEM). The method is described in detail in Journal of Physical Chemistry, 1992, 96, 474-484 incorporated herein by reference.

Cryo-TEM samples were prepared in the controlled environment vitrification system, or CEVS described in detail in Journal of Electron Microscopy, 1988, 10, 87-111. In the CEVS, the temperature was set at 25° and was controlled to within ±0.1°C. Before introducing the sample into the CEVS, the chamber was fitted with porous sponges extending upward from liquid reservoirs. The air inside the chamber was recirculated across the sponges to reduce temperature and composition gradients in the vapor. The high relative humidity within the chamber reduces evaporation of water from the sample and prevents artifacts that result from drying.

Thin films of each sample were formed by placing a 3 μl drop of the sample liquid on a holey polymer support film which had been coated with carbon and mounted on the surface of a standard TEM grid. The drop was blotted with filter paper so that thin (10-500 nm) films of the sample remained, and these spanned the 2-8 μm holes in the support film. The assembly was then vitrified by rapidly plunging it through a synchronous shutter at the bottom of the chamber into liquid ethane at its freezing point.

The vitrified samples were examined at 100-120 kV in the conventional TEM mode of an analytical electron microscope (JEOL Models JEM 100CX and 120CX and Phillips CM12). The cryo transfer holder temperature was maintained below -165°C during imaging. Images were recorded at approximately 4 μm under focus of the microscope objective lens to provide sufficient phase contrast, which is mainly responsible for gradients of optical density in images. The worm-like micelles are identified as randomly orientated rods or curved worm-like shapes of approximately 5nm in diameter and 2 μm in length on the micrographs. Example cryo-TEM images of worm-like micelles can be found in Journal of Physical Chemistry, 1992, 96, 474-484, incorporated herein by reference.

<u>Worm-like micelle phase thickening system test method</u>

The presence of a worm-like micelle thickening phase system in the oxidative hair dye and or bleaching composition according to the present invention can be identified according to the method described below. The following components of the composition are combined in a suitable container at 25°C, at the same levels as used in the final composition:

> all ionic surfactants, all optional amphoteric surfactants if present, all optional non-ionic surfactants if present, all electrolytes which provide counter-ions to the ionic surfactants, all oxidizing agents, all solvents, and water to make the composition up to 100%.

The components are thoroughly mixed for at least 1 hour, after which the composition is filtered to remove any un-dissolved materials. The filtrate is then investigated for the presence of worm-like micelles using the cryo-TEM methodology described hereinabove.

The viscosity of the filtrate is measured using a Brookfield viscometer with cone and plate attachment according to the method described herein. The presence of worm-like micelle thickening phase is established if cryo-TEM micrograph shows at least a portion/section of worm-like micelles, and if the viscosity is at least 1000 cPs.

<u>Viscosty Test method</u>

The viscosity is measured using Brookfield viscometers with cone and plate attachment. For viscosities in the range of 0-12000 cPs the Brookfield DV-11 viscometer with S42 plate is used. 2ml sample of the composition is equilibrated at 26.7°C for three minutes before the readings are taken at 1 rpm. For viscosities in the range of 12,000-50,000 cPs the Brookfield DV-1 viscometer with S52 plate is used. 0.5ml sample of the composition is equilibrated for 1 minute at 26.7°C before the readings are taken at 1 rpm.

## Examples

The following examples illustrate oxidative dye compositions according to the present invention. It is understood that the examples and embodiments described herein are for illustrative purposes only and that various modifications or changes in light thereof will be suggested to one skilled in the art without departing from the scope of the present invention.

Examples 1 - 10 (mixed compositions)

| Ingredient | 1 | 2 | 3 | 4 | 5 |
|---|---|---|---|---|---|
| Ammonium Carbonate | 2 | 7.5 | 4 | 5 | 5 |
| Ammonium Hydrogen Carbonate | - | - | - | - | - |
| Ammonium Carbamate | - | - | - | - | - |
| Sodium Glycinate | 1.0 | 5.0 | 2.0 | 2.5 | 2.5 |
| Cetyltrimethyl Ammonium Chloride | - | - | - | - | - |
| Sodium Lauryl Sulfate | 0.825 | 0.3 | - | 0.825 | 0.825 |
| Sodium Laureth-3 Sulfate | 2.48 | 0.9 | - | 2.48 | 2.48 |
| Sodium Myristoyl Sarcosinate | - | - | - | - | - |
| Sodium Lauryl Phosphate | - | - | - | - | - |
| Sodium Laureth-3 Phosphate | - | - | - | - | - |
| Cocamidopropyl Betaine | 4.95 | 1.8 | - | 4.95 | 4.95 |
| Sodium Lauryl Amphoacetate | - | - | 3.6 | - | - |
| Sodium Cetyl Sulfate (Branched) | - | - | 1.4 | - | - |
| Sodium Cetyl Ether-1 Sulfate (Branched) | - | - | 1.4 | - | - |
| Sodium Chloride | 7.25 | - | 3.0 | 2.75 | 2.75 |
| p-phenylene diamine | 0.1 | | 0.8 | - | 0.1 |
| p-amino phenol | 0.4 | 0.3 | - | 0.3 | 0.4 |
| 2,5-diamino-toluene sulphate | - | 0.1 | - | 0.1 | - |
| m-aminophenol | - | - | 0.2 | - | - |
| Resorcinol | 0.4 | 0.5 | - | 0.5 | 0.4 |
| napthol | - | - | 0.03 | - | - |
| 4-amino-2-hydroxy toluene | 0.3 | 0.2 | - | 0.2 | 0.3 |
| Phenyl methyl pyrazalone | - | - | - | 0.2 | - |
| 1-hydroxyethyl-4,5-diamino pyrazole sulphate | - | - | - | 0.3 | - |
| Basic red 51 | - | 0.1 | - | 0.1 | - |
| Basic yellow 87 | - | 0.2 | - | 0.2 | - |

| | | | | | |
|---|---|---|---|---|---|
| Hydrogen Peroxide (35% active) | 2.85 | 11.0 | 11.0 | 17.14 | 12.85 |
| Amidomethicone(DCAP 6087) | - | - | - | 1.0 | - |
| Polyquaternium-22 (Merquat 295) | - | - | - | - | - |
| Polyquaternium-37 & Mineral oil (Salcare SC95) | - | - | - | - | - |
| Xanthan gum | - | 0.2 | - | - | - |
| EDTA (tetra-sodium salt) | 0.1 | 0.1 | 0.1 | 0.1 | 0.1 |
| Sodium sulphite | 0.1 | 0.1 | 0.1 | 0.1 | 0.1 |
| Ascorbic Acid | 0.1 | 0.1 | 0.1 | 0.1 | 0.1 |
| Propylene Glycol | 0.0 | 0.0 | 0.0 | 0.0 | 3.0 |
| Etidronic Acid | 0.1 | 0.1 | 0.1 | 0.1 | 0.1 |
| pH adjust to pH 9.0 | qs | qs | qs | qs | qs |
| Water | qs | qs | qs | qs | qs |

| **Ingredient** | **6** | **7** | **8** | **9** | **10** |
|---|---|---|---|---|---|
| Ammonium Carbonate | 5 | 10 | - | 5 | 5 |
| Ammonium Hydrogen Carbonate | - | - | 4.0 | | |
| Ammonium Carbamate | - | - | 4.0 | - | - |
| Sodium Glycinate | 2.5 | 10 | 2.5 | - | 2.5 |
| Cetyltrimethyl Ammonium Chloride | - | - | - | - | 8.0 |
| Sodium Lauryl Sulfate | - | - | - | 0.825 | - |
| Sodium Laureth-3 Sulfate | - | - | - | 2.48 | - |
| Sodium Myristoyl Sarcosinate | - | 2.75 | - | - | - |
| Sodium Lauryl Phosphate | - | - | 1.65 | - | - |
| Sodium Laureth-3 Phosphate | - | - | 4.95 | - | - |
| Cocamidopropyl Betaine | - | 2.75 | - | 4.95 | - |
| Sodium Lauryl Amphoacetate | 3.6 | - | 10.8 | - | - |
| Sodium Cetyl Sulfate (Branched) | 1.4 | - | - | - | - |

| | | | | | |
|---|---|---|---|---|---|
| Sodium Cetyl Ether-1 Sulfate (Branched) | 1.4 | - | - | - | - |
| Sodium Chloride | 1.0 | - | - | 5.25 | |
| p-phenylene diamine | 0.8 | - | 0.6 | 0.1 | 0.8 |
| p-amino phenol | - | - | - | 0.4 | - |
| 2,5-diamino-toluene sulphate | - | - | 0.2 | - | - |
| m-aminophenol | 0.2 | - | 0.1 | - | 0.2 |
| Resorcinol | - | - | - | 0.4 | - |
| napthol | 0.03 | - | 0.2 | - | 0.03 |
| 4-amino-2-hydroxy toluene | - | - | - | 0.3 | - |
| Phenyl methyl pyrazalone | - | - | - | - | - |
| 1-hydroxyethyl-4,5-diamino pyrazole sulphate | - | - | - | - | - |
| Basic red 51 | - | - | 0.2 | - | - |
| Basic yellow 87 | - | - | 0.3 | - | - |
| Hydrogen Peroxide (35% active) | 8.6 | 12.85 | 11.0 | 12.85 | 17.14 |
| Amidomethicone (DCAP 6087) | - | - | - | - | - |
| Polyquaternium-22 (Merquat 295) | 0.1 | - | - | 0.1 | - |
| Polyquaternium-37 & Mineral oil (Salcare SC95) | 0.2 | - | - | 0.2 | - |
| Xanthan gum | - | - | - | - | 0.2 |
| EDTA (tetra-sodium salt) | 0.1 | 0.1 | 0.1 | 0.1 | 0.1 |
| Sodium sulphite | 0.1 | 0.1 | 0.1 | 0.1 | 0.1 |
| Ascorbic Acid | 0.1 | 0.1 | 0.1 | 0.1 | 0.1 |
| Propylene Glycol | 0.0 | 0.0 | 0.0 | 0.0 | 0.0 |
| Etidronic Acid | 0.1 | 0.1 | 0.1 | 0.1 | 0.1 |
| pH adjust to pH 9.0 | qs | qs | qs | qs | qs |
| Water | qs | qs | qs | qs | qs |

**Examples 11 - 13 (mixed compositions) and comparative example (mixed composition)**

| Ingredient | 11 | 12 | 13 | Comparative Example 14 |
|---|---|---|---|---|
| Ammonium Hydroxide (29% solution) | 4.5 | - | 4.5 | - |
| Ammonium Carbonate | - | 5.0 | - | 2.2 |
| Sodium Glycinate | - | 2.5 | - | - |
| Sodium Lauryl Sulfate | 0.55 | 0.55 | 0.55 | - |
| Sodium Laureth-3 Sulfate | 1.65 | 1.65 | 1.65 | - |

(continued)

| Ingredient | 11 | 12 | 13 | Comparative Example 14 |
|---|---|---|---|---|
| Cocamidopropyl Betaine | 3.3 | 3.3 | 3.3 | 5.0 |
| Stearalkonium Chloride | - | - | - | 2.0 |
| Sodium Chloride | 10 | 2.75 | - | - |
| Magnesium Chloride | - | - | 1 | - |
| Ceteareth-25 | - | 1.5 | - | - |
| Cetyl Alcohol | - | 1.12 | - | - |
| Stearyl Alcohol | - | 1.12 | - | - |
| p-phenylene diamines | 0.1 | 0.1 | 0.1 | - |
| p-amino phenol | 0.4 | 0.4 | 0.4 | - |
| Resorcinol | 0.4 | 0.4 | 0.4 | - |
| 4-amino-2-hydroxy toluene | 0.3 | 0.3 | 0.3 | - |
| Hydrogen Peroxide (35% active) | 2.85 | 17.14 | 11 | 6 |
| Polyquaternium-22 (Merquat 295) | - | - | 0.1 | - |
| Polyquaternium-37 & Mineral oil (Salcare SC95) | - | - | 0.2 | - |
| Xanthan gum | - | 0.5 | - | - |
| Aculyn 33 | - | - | - | 1.0 |
| EDTA (tetrasodium salt) | 0.1 | 0.1 | 0.1 | - |
| Disodium EDDS | - | - | - | 4.0 |
| Sodium Citrate | - | - | - | 0.4 |
| Sodium sulphite | 0.1 | 0.1 | 0.1 | - |
| Ascorbic Acid | 0.1 | 3.0 | 0.1 | - |
| Propylene Glycol | 0.0 | 0.0 | 0.0 | - |
| Etidronic Acid | 0.1 | 0.1 | 0.1 | - |
| pH | 10 | 9.0 | 10 | 9.0 |
| Water | qs | qs | qs | Qs |

For examples 1-13, using the method to defined hereinabove the viscosity of the mixed systems are 6000 to 10000 cPs. These examples are determined to have a wonn-like micelle thickening phase. Example 14 is a comparative example of a non-thickened worm-like micelle phase. That is, while worm-like micelles are present, on applying the method to determine the presence of a worm like micelle thickening system as described herein, the viscosity was found to be water like and hence not measurable using the method described herein. Thus example 14 is not a worm like micelle thickening system according to the present invention.

**Examples 15-16**

The following hair colouring compositions are prepared (Part A):

| | Ingredient | 15 | 16 |
|---|---|---|---|
| 1 | Ammonium Carbonate | 10 | 15 |
| 2 | Sodium Lauryl Sulfate | 1.65 | - |
| 3 | Sodium Laureth-3 Sulfate | 4.96 | - |

(continued)

| | Ingredient | 15 | 16 |
|---|---|---|---|
| 4 | Cocamidopropyl Betaine | 9.9 | - |
| 5 | Xanthan | - | - |
| 6 | Para-phenylenewianfine | 0.6 | - |
| 7 | Para-aminophenol | - | 0.30 |
| 8 | Meta-aminophenol | 0.2 | - |
| 9 | Resorcinol | - | - |
| 10 | Naphthol | 0.03 | - |
| 11 | Phenyl methyl pyrazalone | 0.2 | - |
| 12 | 1-hydroxyethyl-4-5-diamineo pyrazole | 0.3 | - |
| 13 | 2,5 diaminotolulene sulphate | - | - |
| 14 | 4 amino-2-hydroxytoluene | - | - |
| 15 | EDTA (tetrasodium salt) | 0.1 | 0.1 |
| 16 | Sodium sulphite | 0.1 | 0.1 |
| 17 | Ascorbic Acid | 0.1 | 0.1 |
| 18 | Sodium Glycinate | 5 | 15 |
| 19 | Sodium Chloride | 5.5 | - |
| 20 | pH adjust to pH 9.0 | qs | qs |
| 21 | Water | qs | qs |

The viscosity of the composition of Example 15 and 16 (part A) is below 1000 cPs i.e. it is a thin-thin composition.

The following developer compositions are prepared (Part B):

| | Ingredient | Formulation # | |
|---|---|---|---|
| | | 17 | 18 |
| 1 | Hydrogen peroxide (35%) | 17.0 | 25.71 |
| 2 | Etidronic Acid | 0.2 | 0.2 |
| 3 | Polyquaterniunn-22 (Merquat 295) | 0.1 | - |
| 4 | Polyquaternium-37 & Mineral oil (Salcare SC95) | 0.2 | - |
| 5 | Sodium Lauryl Sulfate | - | 1.65 |
| 6 | Sodium Laureth-3 Sulfate | - | 4.96 |
| 7 | Cocamidopropyl Betaine | - | 9.9 |
| 8 | Xanthan | - | 0.4 |
| 9 | Water | qs | qs |

Part A and Part B are mixed prior to application on hair and the viscosity of the mixed formulations is within the range of 1000 to 60000 cPs.

## Claims

**1.** A hair colouring and or bleaching composition comprising

i) at least one oxidizing agent and
ii) at least one worm-like micelle thickening system comprising

a) from 0.1 to 40.0 % of at least one ionic surfactant and optionally at least one amphoteric surfactant and
b) at least one electrolyte source of counter-ions for said ionic surfactant having a concentration of at least 0.25 mole/kg, wherein the total concentration C of said counter-ions is defined according to the formula:

$$C = \sum M * 10^{(z-1)}$$

wherein M is equal to the molar concentration of each counter ion and z is equal to the charge on each corresponding counter ion.

**2.** A hair colouring and or bleaching composition according to claim 1, wherein said composition comprises less than 10% solvent, preferably less than 8% solvent.

**3.** A hair colouring and or bleaching composition according to claim 1, wherein said ionic surfactant is an anionic surfactant selected from alkyl sulphates, alkyl ether sulphates, alkyl phosphates, alkyl ether phosphates, alkyl glyceryl sulphonates, N-acyl sarcosinates, N acyl taurates, acyl lactylates, carboxyalkyl ethers of alkyl polyglucosides, fatty acid salts, alkyl ether carboxylates and mixtures thereof.

**4.** A hair coloring and or bleaching composition according to claim 1, wherein said ionic surfactant is a cationic surfactant selected from quaternary ammonium salts, amido-amines and mixtures thereof.

**5.** A hair colouring and or bleaching composition according to any one of the preceding claims, wherein said composition further comprises at least one amphoteric or zwitterionic surfactant, preferably selected from alkyl-ampho mono- and di-acetates, alkyliminodiacetates, alkylamidopropyl betaines, alkylamido betaines, alkyl betaines, alkyl dimethyl amine oxides, dihydroxyethyl alkyl amine oxides, alkylamine oxides, dihydroxyethylamine oxides and mixtures thereof.

**6.** A hair colouring and or bleaching composition according to claim 5, wherein said amphoteric surfactant is selected from cocamidopropyl betaine, sodium lauryl amphoacetate or mixtures thereof.

**7.** A hair colouring and or bleaching composition according to claims 1 and 5, wherein said ionic surfactant is selected from N-acyl sarcosinates, alkyl sulphates, alkyl phosphates, alkyl ether sulphates, alkyl ether phosphates and mixtures thereof and said amphoteric or zwitterionic surfactant is selected from cocoamidopropylbetaine, sodium lauryl amphoacetate and mixtures thereof.

**8.** A hair colouring and or bleaching composition according to any one of the preceding claims, wherein said electrolyte as source of counter-ions for said ionic surfactant has a concentration C of from 0.50 mole/kg to 4 mole/kg, preferably from 1 mole/kg to 3 mole/kg.

**9.** A hair colouring and or bleaching composition according to any one of the preceding claims, wherein said electrolyte as a source of counter ions for said ionic surfactant is selected from a source of carbonate ions, a source of ammonium ions, a source of radical scavenger and mixtures thereof

**10.** A hair colouring and or bleaching composition according to claim 9, wherein said source of carbonate ions is selected from ammonium carbonate, ammonium hydrogencarbonate, ammonium carbamate, potassium carbonate, sodium carbonate, sodium hydrogen carbonate, potassium hydrogen carbonate and mixtures thereof.

**11.** A hair colouring and or bleaching composition according to claim 9, wherein said source of ammonium ions is selected from ammonium chloride, ammonium sulphate, ammonium nitrate, ammonium phosphate, ammonium acetate and mixtures thereof.

**12.** A hair colouring and or bleaching composition according to claim 9, wherein said source of radical scavenger is selected from potassium, sodium and ammonium salts of glycine, sarcosine, lysine, serine, glutamic acid, and mixtures thereof.

**13.** A hair colouring and or bleaching composition according to claim 1, wherein said composition has a pH of from 8.4 to 9.5.

**14.** A hair colouring and or bleaching composition according to any one of the preceding claims, wherein said composition has a viscosity of from 1000cPs to 60000 cPs, preferably from 2000cPs to 30000 cPs and most preferably from 3000cPs to 25000 ops.

**15.** A hair colouring and or bleaching composition according to any one of the preceding claims, wherein said composition comprises at least one oxidative dye precursor and or at least one pre-formed dye.

**16.** A hair colouring and or bleaching composition according to any one of the preceding claims, wherein said composition comprises at least one polymer, preferably selected from associative polymers, crosslinked acrylic acid homopolymers, crosslinked copolymers of (meth)acrylic acid and of (C1-C6)alkyl acrylate, nonionic homopolymers and copolymers comprising ethylenically unsaturated monomers of ester and amide type, ammonium acrylate homopolymers and copolymers of ammonium acrylate and of acrylamide or polysaccharides and mixitures thereof.

**17.** A hair colouring and or bleaching kit comprising

i) an individually packaged first oxidising component comprising at least one source of hydrogen peroxide and
ii) an individually packaged second component comprising

a) from 0.1 to 40.0 % of at least one ionic surfactant and
b) at least one electrolyte source of counter-ions for said ionic surfactant,
wherein upon mixing said first and second components i) and ii), the resultant composition mixture comprises at least one worm-like micelle thickening system comprising said ionic surfactant and said electrolyte as source of counter-ions at a concentration C of at least 0.25 mole/kg, where C is defined according to the following formula

$$C = \sum M * 10^{(z-1)}$$

wherein M is equal to the molar concentration of each counter ion and z is equal to the charge on each corresponding counter ion,

**18.** A hair colouring and or bleaching kit comprising

i) an individually packaged first oxidising component comprising

a) at least one source of hydrogen peroxide and
b) from 0.1 to 40.0 % of at least one ionic surfactant and

ii) an individually packaged second component comprising at least one electrolyte source of counter-ions for said ionic surfactant,
wherein upon mixing said first and second components i) and ii), the resultant composition mixture has at least one worm-like micelle thickening system comprising said ionic surfactant and said electrolyte as a source of counter-ions at a concentration C of at least 0.25 mole/kg, where C is defined according to the following formula: $C = \sum M * 10^{(z-1)}$, wherein M is equal to the molar concentration of each counter ion and z is equal to the charge on each corresponding counter ion.

**19.** A hair colouring or bleaching kit according to claims 17 and 18, wherein the viscosity of said second component ii) is less than 1000 cPs and wherein the viscosity of the resultant composition mixture of said first and second components i) and ii), is from 1000cPs to 60000cPs, more preferably from 2000cPs to 30000 cPs.

**20.** A hair colouring and or bleaching kit comprising an individually packaged first component composition and an individually packaged second component composition wherein said first and said second component compositions independently have a viscosity of less than 1000cps and, wherein upon mixing said first and said second component compositions, the resultant composition mixture has a viscosity of from 1000cPs to 60000cPs and wherein said

resultant composition comprises a worm-like micelle phase thickening system and comprises less than 10% of a solvent.

**21.** The use of worm-like micelle systems to thicken hair colouring and or hair bleaching and or hair highlighting compositions.

**Patentansprüche**

**1.** Haarfärbe- und/oder -bleichmittelzusammensetzung, umfassend

I) mindestens ein Oxidationsmittel und
II) mindestens ein Verdickungssystem stäbchenförmiger Mizellen, umfassend

a) zu 0,1 bis 40,0 % mindestens ein ionisches Tensid und wahlweise mindestens ein amphoteres Tensid und
b) mindestens eine elektrolytische Gegenionenquelle für das ionische Tensid mit einer Konzentration von mindestens 0,25 Mol/kg, wobei die Gesamtkonzentration C der Gegenionen gemäß der Formel:

$$C = \sum M * 10^{(z-1)}$$

definiert ist,

wobei M gleich der molaren Konzentration jedes Gegenions ist und z gleich der Ladung jedes entsprechenden Gegenions ist.

**2.** Haarfärbe- und/oder -bleichmittelzusammensetzung nach Anspruch 1, wobei die Zusammensetzung zu weniger als 10 % Lösungsmittel, vorzugsweise zu weniger als 8 % Lösungsmittel umfasst.

**3.** Haarfärbe- und/oder -bleichmittelzusammensetzung nach Anspruch 1, wobei das ionische Tensid ein anionisches Tensid ist, das aus Alkylsulfaten, Alkylethersulfaten, Alkylphosphaten, Alkyletherphosphaten, Alkylglycerylsulfonaten, N-Acylsarcosinaten, N-Acyltauraten, Acyllactylaten, Carboxyalkylethern von Alkylpolyglucosiden, Fettsäuresalzen, Alkylethercarboxylaten und Mischungen davon ausgewählt ist.

**4.** Haarfärbe- und/oder -bleichmittelzusammensetzung nach Anspruch 1, wobei das ionische Tensid ein kationisches Tensid ist, das aus quartären Ammoniumsalzen, Amidoaminen und Mischungen davon ausgewählt ist.

**5.** Haarfärbe- und/oder -bleichmittelzusammensetzung nach einem der vorstehenden Ansprüche, wobei die Zusammensetzung ferner mindestens ein amphoteres oder zwitterionisches Tensid umfasst, das vorzugsweise aus amphoteren Alkylmono- und -diacetaten, Alkyliminodiacetaten, Alkylamidopropylbetainen, Alkylamidobetainen, Alkylbetainen, Alkyldimethylaminoxiden, Dihydroxyethylalkylaminoxiden, Alkylaminoxiden, Dihydroxyethylaminoxiden und Mischungen davon ausgewählt ist.

**6.** Haarfärbe- und/oder -bleichmittelzusammensetzung nach Anspruch 5, wobei das amphotere Tensid aus Cocoamidopropylbetain, Natriumlaurylamphoacetat oder Mischungen davon ausgewählt ist.

**7.** Haarfärbe- und/oder -bleichmittelzusammensetzung nach Anspruch 1 und 5, wobei das ionische Tensid aus N-Acylsarcosinaten, Alkylsulfaten, Alkylphosphaten, Alkylethersulfaten, Alkyletherphosphaten und Mischungen davon ausgewählt ist und das amphotere oder zwitterionische Tensid aus Cocoamidopropylbetain, Natriumlaurylamphoacetat und Mischungen davon ausgewählt ist.

**8.** Haarfärbe- und/oder -bleichmittelzusammensetzung nach einem der vorstehenden Ansprüche, wobei der Elektrolyt als Gegenionenquelle für das ionische Tensid eine Konzentration C von 0,50 Mol/kg bis 4 Mol/kg, vorzugsweise von 1 Mol/kg bis 3 Mol/kg aufweist.

**9.** Haarfärbe- und/oder -bleichmittelzusammensetzung nach einem der vorstehenden Ansprüche, wobei der Elektrolyt als Gegenionenquelle für das ionische Tensid aus einer Carbonationenquelle, einer Ammoniumionenquelle, einer

Radikalfängerquelle und Mischungen davon ausgewählt ist.

**10.** Haarfärbe- und/oder -bleichmittelzusammensetzung nach Anspruch 9, wobei die Carbonationenquelle aus Ammoniumcarbonat, Ammoniumhydrogencarbonat, Ammoniumcarbamat, Kaliumcarbonat, Natriumcarbonat, Natriumhydrogencarbonat, Kaliumhydrogencarbonat und Mischungen davon ausgewählt ist.

**11.** Haarfärbe- und/oder -bleichmittelzusammensetzung nach Anspruch 9, wobei die Ammoniumionenquelle aus Ammoniumchlorid, Ammoniumsulfat, Ammoniumnitrat, Ammoniumphosphat, Ammoniumacetat und Mischungen davon ausgewählt ist.

**12.** Haarfärbe- und/oder -bleichmittelzusammensetzung nach Anspruch 9, wobei die Radikalfängerquelle aus Kalium-, Natrium- und Ammoniumsalzen von Glycin, Sarcosin, Lysin, Serin, Glutaminsäure und Mischungen davon ausgewählt ist.

**13.** Haarfärbe- und/oder -bleichmittelzusammensetzung nach Anspruch 1, wobei die Zusammensetzung einen pH-Wert von 8,4 bis 9,5 aufweist.

**14.** Haarfärbe- und/oder -bleichmittelzusammensetzung nach einem der vorstehenden Ansprüche, wobei die Zusammensetzung eine Viskosität von 1000 cP bis 60000 cP, vorzugsweise von 2000 cP bis 30000 cP und am meisten bevorzugt von 3000 cP bis 25000 cP aufweist.

**15.** Haarfärbe- und/oder -bleichmittelzusammensetzung nach einem der vorstehenden Ansprüche, wobei die Zusammensetzung mindestens einen Vorläufer von oxidativem Farbstoff und/oder mindestens einen vorgebildeten Farbstoff umfasst.

**16.** Haarfärbe- und/oder -bleichmittelzusammensetzung nach einem der vorstehenden Ansprüche, wobei die Zusammensetzung mindestens ein Polymer umfasst, das vorzugsweise aus assoziativen Polymeren, vernetzten Acrylsäure-Homopolymeren, vernetzten Copolymeren von (Meth)acrylsäure und von (C1-C6)-Alkylacrylat, nichtionischen Homopolymeren und Copolymeren, die ethylenisch ungesättigte Monomere vom Ester- und Amidtyp umfassen, Ammoniumacrylat-Homopolymeren und Copolymeren von Ammoniumacrylat und von Acrylamid oder Polysacchariden und Mischungen davon ausgewählt ist.

**17.** Haarfärbe- und/oder -bleichset, umfassend

I) einen einzeln verpackten ersten Oxidierungsbestandteil, umfassend mindestens eine Wasserstoffperoxidquelle, und
II) einen einzeln verpackten zweiten Bestandteil, umfassend

a) zu 0,1 bis 40,0 % mindestens ein ionisches Tensid und
b) mindestens eine elektrolytische Gegenionenquelle für das ionische Tensid,

wobei nach dem Mischen des ersten und zweiten Bestandteils I) und II) die resultierende Zusammensetzungsmischung mindestens ein Verdickungssystem stäbchenförmiger Mizellen umfasst, umfassend das ionische Tensid und das Elektrolyt als Gegenionenquelle in einer Konzentration C von mindestens 0,25 Mol/kg,
wobei C gemäß folgender Formel definiert ist:

$$C = \sum M * 10^{(z-1)}$$

wobei M gleich der molaren Konzentration jedes Gegenions ist und z gleich der Ladung jedes entsprechenden Gegenions ist,

**18.** Haarfärbe- und/oder -bleichset, umfassend

I) einen einzeln verpackten ersten Oxidierungsbestandteil, umfassend

a) mindestens eine Wasserstoffperoxidquelle und

b) zu 0,1 bis 40,0 % mindestens ein ionisches Tensid, und

II) einen einzeln verpackten zweiten Bestandteil, umfassend mindestens eine elektrolytische Gegenionenquelle für das ionische Tensid,

wobei nach dem Mischen des ersten und zweiten Bestandteils I) und II) die resultierende Zusammensetzungsmischung mindestens ein Verdickungssystem stäbchenförmiger Mizellen aufweist, umfassend das ionische Tensid und das Elektrolyt als Gegenionenquelle in einer Konzentration C von mindestens 0,25 Mol/kg, wobei C gemäß folgender Formel definiert ist: $C = \sum M^* 10^{(z-1)}$, wobei M gleich der molaren Konzentration jedes Gegenions ist und z gleich der Ladung jedes entsprechenden Gegenions ist.

**19.** Haarfärbe- oder -bleichset nach Anspruch 17 und 18, wobei die Viskosität des zweiten Bestandteils II) weniger als 1000 cP beträgt und wobei die Viskosität der resultierenden Zusammensetzungsmischung des ersten und zweiten Bestandteils I) und II) 1000 cP bis 60000 cP, bevorzugter 2000 cP bis 30000 cP beträgt.

**20.** Haarfärbe- und/oder -bleichset, umfassend eine einzeln verpackte Zusammensetzung eines ersten Bestandteils und eine einzeln verpackte Zusammensetzung eines zweiten Bestandteils, wobei die Zusammensetzungen des ersten und des zweiten Bestandteils unabhängig voneinander eine Viskosität von weniger als 1000 cP aufweisen und wobei nach dem Mischen der Zusammensetzungen des ersten und des zweiten Bestandteils die resultierende Zusammensetzungsmischung eine Viskosität von 1000 cP bis 60000 cP aufweist und wobei die resultierende Zusammensetzung ein Verdickungssystem mit einer Phase stäbchenförmiger Mizellen umfasst und zu weniger als 10 % Lösungsmittel umfasst.

**21.** Verwendung von Systemen stäbchenförmiger Mizellen zum Verdicken von Haarfärbe- und/oder Haarbleichmittel- und/oder Haarsträhnchenzusammensetzungen.

## Revendications

**1.** Composition de coloration et/ou d'éclaircissement capillaire comprenant

i) au moins un agent oxydant et
ii) au moins un système épaississant micellaire de type ver comprenant

a) de 0,1 à 40,0 % d'au moins un agent tensioactif ionique et facultativement au moins un agent tensioactif amphotère et
b) au moins une source électrolyte de contre-ions pour ledit agent tensioactif ionique ayant une concentration d'au moins 0,25 mole/kg, dans laquelle la concentration totale C desdits contre-ions est définie selon la formule : $C = \sum M^* 10^{(z-1)}$
dans laquelle M est égal à la concentration molaire de chaque contre-ion et z est égal à la charge sur chaque contre-ion correspondant.

**2.** Composition de coloration et/ou d'éclaircissement capillaire selon la revendication 1, où ladite composition comprend moins de 10 % de solvant, de préférence moins de 8 % de solvant.

**3.** Composition de coloration et/ou d'éclaircissement capillaire selon la revendication 1, dans laquelle ledit agent tensioactif ionique est un agent tensioactif anionique choisi parmi les sulfates d'alkyle, les sulfates d'alkyléther, les phosphates d'alkyle, les phosphates d'alkyléther, les sulfonates d'alkyl glycéryle, les sarcosinates de N-acyle, les taurates de N-acyle, les lactylates d'acyle, les éthers carboxyalkyliques d'alkyl-polyglucosides, les sels d'acide gras, les carboxylates d'alkyléther et leurs mélanges.

**4.** Composition de coloration et/ou d'éclaircissement capillaire selon la revendication 1, dans laquelle ledit agent tensioactif ionique est un agent tensioactif cationique choisi parmi les sels d'ammonium quaternaire, les amido-amines et leurs mélanges.

**5.** Composition de coloration et/ou d'éclaircissement capillaire selon l'une quelconque des revendications précédentes, où ladite composition comprend en outre au moins un agent tensioactif amphotère ou zwittérionique, choisi de préférence parmi les alkyl-ampho mono- et di-acétates, les alkyliminodiacétates, les alkylamidopropyl bétaïnes, les

alkylamido bétaïnes, les alkyl bétaïnes, les oxydes d'alkyl diméthylamine, les oxydes de dihydroxyéthyl alkylamine, les oxydes d'alkylamine, les oxydes de dihydroxyéthylamine et leurs mélanges.

**6.** Composition de coloration et/ou d'éclaircissement capillaire selon la revendication 5, dans laquelle ledit agent tensioactif amphotère est choisi parmi la cocamidopropyl bétaïne, le sodium-lauryl ampho-acétate ou leurs mélanges.

**7.** Composition de coloration et/ou d'éclaircissement capillaire selon les revendications 1 et 5, dans laquelle ledit agent tensioactif ionique est choisi parmi les sarcosinates de N-acyle, les sulfates d'alkyle, les phosphates d'alkyle, les sulfates d'alkyléther, les phosphates d'alkyléther et leurs mélanges et ledit agent tensioactif amphotère ou zwittérionique est choisi parmi la cocoamidopropyl bétaïne, l'ampho-acétate de sodium-lauryle et leurs mélanges.

**8.** Composition de coloration et/ou d'éclaircissement capillaire selon l'une quelconque des revendications précédentes, dans laquelle ledit électrolyte en tant que source de contre-ions pour ledit agent tensioactif ionique a une concentration C allant de 0,50 mole/kg à 4 moles/kg, de préférence de 1 mole/kg à 3 moles/kg.

**9.** Composition de coloration et/ou d'éclaircissement capillaire selon l'une quelconque des revendications précédentes, dans laquelle ledit électrolyte en tant que source de contre-ions pour ledit agent tensioactif ionique est choisi parmi une source d'ions carbonate, une source d'ions ammonium, une source d'agent anti-radicaux libres et leurs mélanges.

**10.** Composition de coloration et/ou d'éclaircissement capillaire selon la revendication 9, dans laquelle ladite source d'ions carbonate est choisie parmi le carbonate d'ammonium, le bicarbonate d'ammonium, le carbamate d'ammonium, le carbonate de potassium, le carbonate de sodium, le bicarbonate de sodium, le bicarbonate de potassium et leurs mélanges.

**11.** Composition de coloration et/ou d'éclaircissement capillaire selon la revendication 9, dans laquelle ladite source d'ions ammonium est choisie parmi le chlorure d'ammonium, le sulfate d'ammonium, le nitrate d'ammonium, le phosphate d'ammonium, l'acétate d'ammonium et leurs mélanges.

**12.** Composition de coloration et/ou d'éclaircissement capillaire selon la revendication 9, dans laquelle ladite source d'agent anti-radicaux libres est choisie parmi les sels de potassium, sodium et ammonium de glycine, sarcosine, lysine, sérine, acide glutamique, et leurs mélanges.

**13.** Composition de coloration et/ou d'éclaircissement capillaire selon la revendication 1, où ladite composition a un pH allant de 8,4 à 9,5.

**14.** Composition de coloration et/ou d'éclaircissement capillaire selon l'une quelconque des revendications précédentes, où ladite composition a une viscosité allant de 1 000 cP à 60 000 cP, de préférence de 2 000 cP à 30 000 cP et le plus préférablement de 3 000 cP à 25 000 cP.

**15.** Composition de coloration et/ou d'éclaircissement capillaire selon l'une quelconque des revendications précédentes, où ladite composition après mélange comprend au moins un précurseur de teinture oxydative ou/et au moins une teinture préformée.

**16.** Composition de coloration et/ou d'éclaircissement capillaire selon l'une quelconque des revendications précédentes, où ladite composition comprend au moins un polymère, choisi de préférence parmi les polymères associatifs, les homopolymères d'acide acrylique réticulés, les copolymères réticulés d'acide (méth)acrylique et d'acrylate d'alkyle en (C1 à C6), des homopolymères et copolymères non ioniques comprenant des monomères éthyléniquement insaturés de type ester et amide, des homopolymères et copolymères d'acrylate d'ammonium et d'acrylamide ou des polysaccharides et leurs mélanges.

**17.** Trousse de coloration et/ou d'éclaircissement capillaire comprenant

i) un premier composant oxydant conditionné individuellement comprenant au moins une source de peroxyde d'hydrogène et
ii) un deuxième composant conditionné individuellement comprenant

a) de 0,1 à 40,0 % d'au moins un agent tensioactif ionique et
b) au moins une source électrolyte de contre-ions pour ledit agent tensioactif ionique,

dans laquelle lors d'un mélange desdits premier et deuxième composants i) et ii), le mélange de compositions résultant comprend au moins un système épaississant micellaire de type ver comprenant ledit agent tensioactif ionique et ledit électrolyte en tant que source de contre-ions à une concentration C d'au moins 0,25 mole/kg, où C est défini selon la formule suivante $C = \sum M*10^{(z-1)}$ dans laquelle M est égal à la concentration molaire de chaque contre-ion et z est égal à la charge sur chaque contre-ion correspondant.

**18.** Trousse de coloration et/ou d'éclaircissement capillaire comprenant

i) un premier composant oxydant conditionné individuellement comprenant

a) au moins une source de peroxyde d'hydrogène et
b) de 0,1 à 40,0 % d'au moins un agent tensioactif ionique et

ii) un deuxième composant conditionné individuellement comprenant au moins une source électrolyte de contre-ions pour ledit agent tensioactif ionique,
dans laquelle lors d'un mélange desdits premier et deuxième composants i) et ii), le mélange de compositions résultant a au moins un système épaississant micellaire de type ver comprenant ledit agent tensioactif ionique et ledit électrolyte en tant que source de contre-ions à une concentration C d'au moins 0,25 mole/kg, où C est défini selon la formule suivante : $C = \sum M*10^{(z-1)}$, dans laquelle M est égal à la concentration molaire de chaque contre-ion et z est égal à la charge sur chaque contre-ion correspondant.

**19.** Trousse de coloration ou d'éclaircissement capillaire selon les revendications 17 et 18, dans laquelle la viscosité dudit deuxième composant ii) est inférieure à 1000 cP et dans laquelle la viscosité du mélange de compositions résultant desdits premier et deuxième composants i) et ii), va de 1 000 cP à 60 000 cP, plus préférablement de 2 000 cP à 30 000 cP.

**20.** Trousse de coloration et/ou d'éclaircissement capillaire comprenant une première composition de composants conditionnée individuellement et une deuxième composition de composants conditionnée individuellement dans laquelle ladite première et ladite deuxième compositions de composants ont indépendamment une viscosité inférieure à 1 000 cP et, dans laquelle lors d'un mélange de ladite première et ladite deuxième compositions de composants, le mélange de compositions résultant a une viscosité allant de 1 000 cP à 60 000 cP et dans laquelle ladite composition résultante comprend un système épaississant à phase micellaire de type ver et comprend moins de 10 % d'un solvant.

**21.** Utilisation de systèmes micellaires de type ver pour épaissir des compositions de coloration capillaire et/ou d'éclaircissement capillaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 435012 A
- EP 1106166 A
- US 20040083557 A
- WO 04014328 A
- US 20040098814 A
- US 20040098816 A
- EP 1484047 A
- EP 148447 A
- EP 1047375 A
- US 20040053797 A1
- EP 0530708 A
- EP 070160 B1
- US 2528378 A
- US 2781354 A


### Non-patent literature cited in the description


- *Advances in Colloid and interface Science,* 1982, vol. 17, 275-298
- *ADVANCES IN COLLOID AND INTERFACE SCIENCE,* 1986, vol. 26, 177-203
- *Journal of Physical Chemistry,* 22 April 1976, vol. 80 (9), 905-922
- *Journal of Physical Chemistry,* 1988, vol. 92, 4712-4719
- *Langmuir,* 1992, vol. 8, 2140-2146
- *Journal of Physical Chemistry,* 1992, vol. 96, 474-484
- **H Rible.** pH Buffer Theory - A New Approach. John Wiley & Sons, 1996
- *Biochim. Biophysics,* 1989, vol. 988, 221-256
- **M. R. Porter.** Handbook of Surfactants. Blackie & Son, 1991, 116-178
- **Sagarin.** Cosmetic Science and Technology. Interscience, Special Edn, vol. 2, 308-310
- **AE Martell ; RM Smith.** Critical Stability Constants. Plenum Press, 1974, vol. 1
- **AE Martell ; RD Hancock.** Metal Complexes in Aqueous Solution. Plenum Press, 1996
- **Jerry March.** Advanced Organic Chemistry - Reactions, Mechanisms and Structure. 2001, 368-375
- *Journal of Electron Microscopy,* 1988, vol. 10, 87-111
- *Journal of Physical Chemistry,* vol. 96, 474-484